# EUROPEAN PATENT SPECIFICATION

(11) **EP 2 694 079 B1**
(45) Date of publication and mention of the grant of the patent: **17.01.2018**
(21) Application number: 12767243.4
(22) Date of filing: 06.04.2012
(51) Int. Cl.: A61K 31/765, C08G 67/04, C08L 73/02

(54) **POLYMERS AND METHODS FOR THE TREATMENT OF PAIN**
POLYMERE UND VERFAHREN ZUR SCHMERZBEHANDLUNG
POLYMÈRES ET PROCÉDÉS POUR LE TRAITEMENT DE LA DOULEUR

(30) Priority: 06.04.2011 US 201161472526 P
(43) Date of publication of application: 12.02.2014
(73) Proprietor: Rutgers, The State University of New Jersey, New Brunswick, NJ 08903 (US); Uhrich, Kathryn E., New Brunswick, New Jersey 08903 (US); Rosario-Melendez, Roselin, New Brunswick, New Jersey 08903 (US)
(72) Inventor: UHRICH, Kathryn E., New Brunswick, New Jersey 08903 (US); ROSARIO-MELENDEZ, Roselin, New Brunswick, New Jersey 08903 (US)
(74) Representative: Office Freylinger
(86) International application number: PCT/US2012/032548
(87) International publication number: WO 2012/139015

(56) References cited:
- WO-A1-99/12990
- WO-A1-2004/039355
- WO-A2-02/09767
- WO-A2-02/09768
- CHAFI N ET AL: "Dosage form with drug attached to polymer (polyanhydride) dispersed in a Eudragit matrix: preparation and release of drug in gastric liquid", INTERNATIONAL JOURNAL OF PHARMACEUTICS, ELSEVIER BV, NL, vol. 45, no. 3, 1 August 1988 (1988-08-01) , pages 229-236, XP023856537, ISSN: 0378-5173, DOI: 10.1016/0378-5173(88)90292-X [retrieved on 1988-08-01]
- MAHKAM M ET AL: "Preparation of new biodegradable polyurethanes as a therapeutic agent", POLYMER DEGRADATION AND STABILITY, BARKING, GB, vol. 80, no. 2, 1 January 2003 (2003-01-01), pages 199-202, XP004414747, ISSN: 0141-3910, DOI: 10.1016/S0141-3910(02)00388-9
- ROBERT C SCHMELTZER ET AL: "Synthesis and characterization of antiseptic-based poly(anhydride-esters)", POLYMER BULLETIN, SPRINGER, BERLIN, DE, vol. 57, no. 3, 21 April 2006 (2006-04-21) , pages 281-291, XP019423512, ISSN: 1436-2449, DOI: 10.1007/S00289-006-0561-Z
- ROSARIO-MELENDEZ ROSELIN ET AL: "Synthesis, characterization, and in vitro studies of a morphine-based poly(anhydride-ester)", AMERICAN CHEMICAL SOCIETY. ABSTRACTS OF PAPERS (AT THE NATIONAL MEETING), AMERICAN CHEMICAL SOCIETY, US, vol. 242, 1 August 2011 (2011-08-01), page 258, XP009181244, ISSN: 0065-7727
- ROSELIN ROSARIO-MELÉNDEZ ET AL: "PolyMorphine: An innovative biodegradable polymer drug for extended pain relief", JOURNAL OF CONTROLLED RELEASE, vol. 162, no. 3, 1 September 2012 (2012-09-01), pages 538-544, XP055159880, ISSN: 0168-3659, DOI: 10.1016/j.jconrel.2012.07.033
- CHAFI: 'Dosage form with drug attached to polymer (polyanhydride) dispersed in a Eudragit matrix: preparation and release of drug in gastric liquid.' INTERNATIONAL JOURNAL OF PHARMACEUTICS vol. 45, 1988, pages 229 - 236, XP023856537

## Description

### Background of the Invention

Approximately 75 million Americans experience chronic or acute pain. Opiates are the drugs of choice for the treatment of moderate to severe acute and chronic pain and morphine has been the most important and widely used of these drugs. Its short half-life in plasma of 1.7 to 4.5 hours, its analgesic effect that lasts 4 to 6 hours, and the tendency of people to develop tolerance to the drug leads to frequent dosing (every 3 to 4 hours) and patient discomfort. Additionally, these drugs are known to be addictive and are often abused.

Propionic acid derivative non-steroidal anti-inflammatories (NSAIDs), which are over the counter analgesics, are also used to treat pain, as well as fever and inflammation. The major disadvantage of these drugs is their tendency to induce gastric and intestinal erosion, bleeding and ulceration.

Accordingly, new methods and compositions to treat pain are needed. WO2004/039355 discloses a biocompatible, biodegradable polymer incorporating an NSAID in the polymer backbone and compounded in microspheres which also contain morphine. Chafi et al.. Int. J. Pharm. 45(1988) 229-236, discloses a polyanhydride having attached pendant residues of a NSAID. WO02/09768 discloses a polyester polymer having morphine incorporated into its backbone.

### Summary of the Invention

Applicant has discovered new anhydride polymers, new pharmaceutical compositions comprising said anhydride polymers, useful in particular to treat chronic and acute pain. This discovery may increase specificity through localized drug release, eliminate the requirements for frequent dosing by prolonging the release, delay the development of opiate resistance, prevent drug abuse since the drug is not immediately available, or reduce the occurrence of undesired side-effects.

Accordingly, certain embodiments of the present invention provide an anhydride polymer comprising a biodegradable backbone that comprises one or more groups that will provide morphine upon hydrolysis of the polymer and one or more pendant residues of a non-steroidal anti-inflammatory.

Certain embodiments of the present invention also provide a microsphere that comprises an anhydride polymer as described herein.

Certain embodiments of the present invention provide a pharmaceutical composition comprising an anhydride polymer as described herein and a pharmaceutically acceptable carrier.

The present document also describes a method to treat pain in a mammal, comprising administering a first polymer comprising repeating units that form a biodegradable backbone, wherein morphine is incorporated into the backbone, to the mammal.

The present document also describes a method to treat pain in a mammal, comprising administering an anhydride polymer as described herein to the mammal.

The present document also describes a method to treat pain in a mammal, comprising administering a first polymer comprising a backbone which comprises one or more groups in the backbone that will provide morphine upon hydrolysis of the polymer, and a second polymer, as described herein, to the mammal.

Certain embodiments of the present invention provide an anhydride polymer as described herein for use in medical treatment.

Certain embodiments of the present invention provide an anhydride polymer as described herein for use in treating pain.

### Brief Description of the Figures

Figure 1. (A) Structure of SA-adipic polymer and SA-diethylmalonic polymer (B) SEM images of SA-adipic polymer microspheres (a) and SA-diethylmalonic polymer microspheres (b) before hydrolytic degradation. (C) Calibration curve used to calculate the concentration of SA released daily. (D) *In vitro* hydrolytic degradation profiles of SA-adipic (1a) and SA-DEM (1c) microspheres. (E) *In vitro* hydrolytic degradation profiles of microspheres with physical admixtures (*see* Table 1).
Figure 2. Cell counts of L929 fibroblast cells grown in media containing the 0.01 mg/mL polymer (top) and *in vitro* hydrolytic degradation profiles of radiation exposed samples (bottom).
Figure 3. ¹H-NMR spectra of compounds **5a** and **6a** showing the presence and disappearance of the benzyl protecting groups.
Figure 4. ¹³C-NMR spectra of morphine **1**, diacid **3**, and PolyMorphine **5**, showing the preservation of the chemical integrity of the drug; key peaks for the nitrogen-containing ring are indicated.
Figure 5. Infrared spectra of PolyMorphine 5 and diacid 3, key stretch bands for OH acid, C=O acid, C=O ester, and C=O anhydride are indicated.
Figure 6. (Top) Hydrolytic degradation scheme of PolyMorphine (5) and structure of monoacid (7). (Bottom) Chromatograms showing the *in vitro* degradation of diacid (3) into monoacid (6) and free morphine (1) at different time points (2 h, 5 h, 10 h, 1 d, 5 d, 10 d, 15 d, 20 d, 25 d, and 30 d).
Figure 7. *In vitro* cell cytocompatibility of diacid (3) and PolyMorphine (5). (A) Cell viability of the positive control (fibroblasts with cell culture media only), 3 (at 0.10 mg/mL), and 5 (at 0.10 mg/mL), no statistical differences at 95 % confidence level between the samples containing 3 and 5 and the positive control; Fluorescent microscopy images of: (B) positive control, (C) negative control (fibroblasts with cell culture media and 5 % ethanol), (D) diacid 3, and (E) 5.
Figure 8. (A) TFL results at 0.5-24 h post-administration. (B) TFL results from day 1 through day 14 (vertical arrows indicate the days that animals received acute morphine challenge to evaluate morphine tolerance development). PolyMorphine provides extended analgesia compared with free morphine. Data are shown as mean ± standard error of mean. N = 30 for each time point prior to and including day 3. N = 15 after day 3.
Figure 9. TFL results to acute morphine challenge (10 mg/kg, i.p.) (A) 3 days (B) 14 days after the initial administration. N = 15 for all groups. Animals retain full responsiveness to acute morphine challenge, regardless of whether they received free morphine or PolyMorphine. If the animals become tolerant to morphine, it is expected that they would be non-responsive or would flick their tails in less than 30 s (cutoff time) when their tails are immersed in the hot water.
Figure 10. Scanning electron microscopy images of microspheres generated from polymer 5. (A) 995x magnification; (B) 2,520x magnification.

### Detailed Description

Certain embodiments of the present invention provide an anhydride polymer comprising a biodegradable backbone that comprises one or more groups that will provide morphine upon hydrolysis of the polymer and one or more pendant residues of a non-steroidal anti-inflammatory.

As used herein, an "anhydride polymer" is a polymer that has anhydride bonds in the backbone of the polymer. In one embodiment the anhydride polymer is formed from monomer units that react to provide the anhydride bonds. NSAIDs are incorporated into the anhydride polymers of the invention as pendant groups that are not part of the backbone of the polymer. As such, a tracing of the chain of atoms that form the backbone of the anhydride polymer would not include the atoms of the residues of the NSAIDs. In certain embodiments of the invention, the pendant groups can be considered to be sidechains of the polymer. NSAIDs can be attached to the remainder of the anhydride polymer of the invention through labile (*e.g*. anhydride, ester, amide or thioester linkages) bonds, that allow for release of the NSAIDs upon degradation (*e.g*. hydrolysis).

In certain embodiments, an anhydride polymer as described herein comprises repeating units that form the biodegradable backbone, wherein each repeating unit comprises one or more pendant residues of the non-steroidal anti-inflammatory.

In certain embodiments, an anhydride polymer as described herein comprises repeating units that form the biodegradable backbone, wherein each repeating unit comprises 1, 2, 3, 4, 5, 6, 7, 8, or 9 pendant residues of the non-steroidal anti-inflammatory.

In certain embodiments, each repeating unit comprises 2 pendant residues of the non-steroidal anti-inflammatory.

In certain embodiments, an anhydride polymer as described herein comprises one or more groups of formula (I):

-C(=O)-A-C(=O)-O- (I)

wherein A is a C₁-C₈ methylene chain that is covalently linked to one or more residues of a non-steroidal anti-inflammatory.

In certain embodiments, the C₁-C₈ methylene chain is covalently linked to the one or more residues of the non-steroidal anti-inflammatory through an amine, ester, amide, sulfide, or ether linkage.

In certain embodiments, an anhydride polymer as described herein comprises one or more groups of formula (Ia):

-C(=O)-[CH(B)]₁₋₈-C(=O)-O- (Ia)

wherein each B is independently a residue of a non-steroidal anti-inflammatory.

In certain embodiments, an anhydride polymer as described herein comprises one or more groups of formula (II): 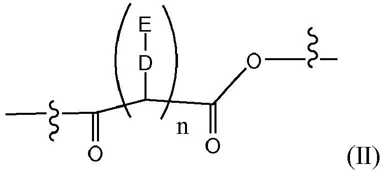 wherein each D is a direct bond, or an amine, ester, amide, sulfide, or ether linkage; each E is independently a residue that will release a non-steroidal anti-inflammatory agent upon hydrolysis of the polymer; and n is 1, 2, 3, 4, 5, 6, 7, 8, or 9.

In certain embodiments, n is 2.

In certain embodiments, D is -O-.

In certain embodiments, an anhydride polymer as described herein comprises one or more groups of formula (IIa): 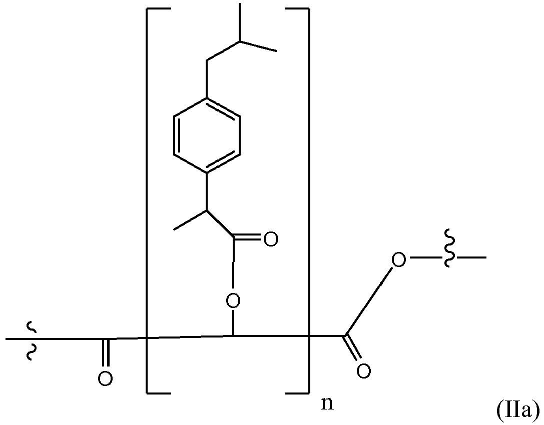 wherein n is 1, 2, 3, 4, 5, 6, 7, 8, or 9.

In certain embodiments, n is 2.

In certain embodiments, an anhydride polymer as described herein comprises one or more groups of formula (IIb): 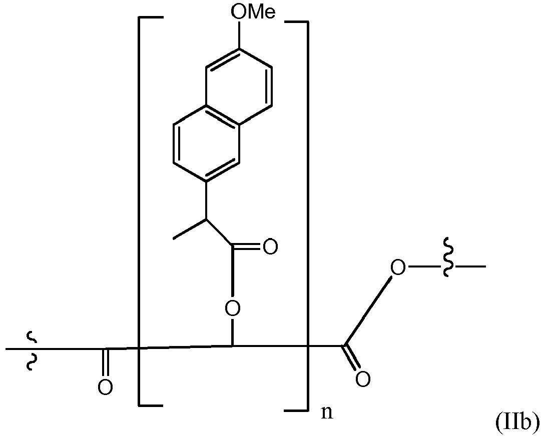 wherein n is 1, 2, 3, 4, 5, 6, 7, 8, or 9.

In certain embodiments, n is 2.

In certain embodiments, an anhydride polymer as described herein comprises two or more repeating groups of formula (II): 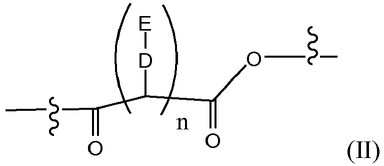 wherein each D is a direct bond, or an amine, ester, amide, sulfide, or ether linkage; each E is independently a residue that will release a non-steroidal anti-inflammatory agent upon hydrolysis of the anhydride polymer; and n is 1, 2, 3, 4, 5, 6, 7, 8, or 9. In certain embodiments, n is 2.

In certain embodiments, an anhydride polymer as described herein comprises 2-200 repeating groups of formula (II). In certain embodiments, an anhydride polymer as described herein comprises about 2-150 repeating groups of formula (II). In certain embodiments, an anhydride polymer as described herein comprises about 2-100 repeating groups of formula (II). In certain embodiments, an anhydride polymer as described herein comprises about 2-75 repeating groups of formula (II). In certain embodiments, an anhydride polymer as described herein comprises about 25-75 repeating groups of formula (II). In certain embodiments, an anhydride polymer as described herein comprises about 40-60 repeating groups of formula (II).

In certain embodiments, an anhydride polymer as described herein comprises at least 2, 3, 4, 5, 6, 7, 8, or 9 repeating groups of formula (II).

In certain embodiments, an anhydride polymer as described herein comprises two or more repeating groups of formula (IIa): 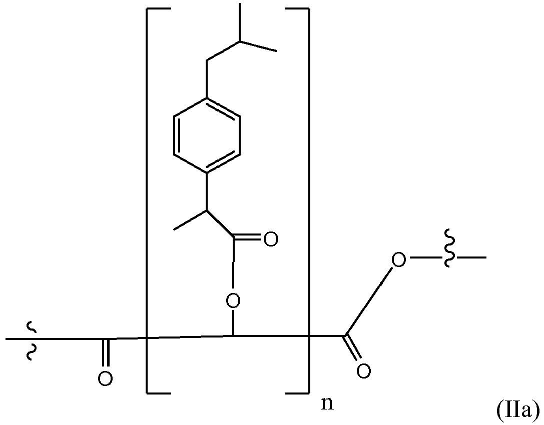 wherein n is 1, 2, 3, 4, 5, 6, 7, 8, or 9.

In certain embodiments, n is 2.

In certain embodiments, an anhydride polymer as described herein comprises two or more repeating groups of formula (IIb): 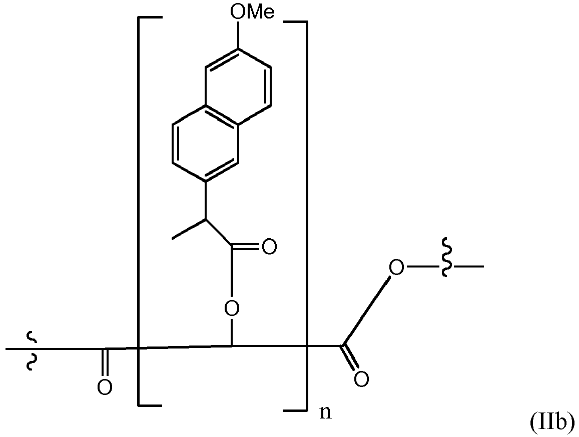 wherein n is 1, 2, 3, 4, 5, 6, 7, 8, or 9.

In certain embodiments, n is 2.

In certain embodiments, an anhydride polymer as described herein comprises 2-200 repeating groups of formula (IIa) or (IIb). In certain embodiments, an anhydride polymer as described herein comprises about 2-150 repeating groups of formula (IIa) or (IIb). In certain embodiments, an anhydride polymer as described herein comprises about 2-100 repeating groups of formula (IIa) or (IIb). In certain embodiments, an anhydride polymer as described herein comprises about 2-75 repeating groups of formula (IIa) or (IIb). In certain embodiments, an anhydride polymer as described herein comprises about 25-75 repeating groups of formula (IIa) or (IIb). In certain embodiments, an anhydride polymer as described herein comprises about 40-60 repeating groups of formula (IIa) or (IIb).

In certain embodiments, an anhydride polymer as described herein comprises at least 2, 3, 4, 5, 6, 7, 8, or 9 repeating groups of formula (IIa) or (IIb).

Non-steroidal anti-inflammatory agents (NSAIDs) are a well known class of drugs that includes, for example, ibuprofen, naproxen, fenoprofen, ketoprofen, flurbiprofen, suprofen, benoxaprofen, indoprofen, pirprofen, carprofen, loxoprofen, pranoprofen, alminoprofen, salicylic acid, diflunisal, salsalate, oxaprozin, indomethacin, sulindac, etodolac, ketorolac, diclofenac, piroxicam, meloxicam, tenoxican, lornoxicam, isoxicam, mefenamic acid, meclofenamic acid, flufenamic acid, tolfenamic acid, lumiracoxib and licofelone.

Accordingly, in certain embodiments, the non-steroidal anti-inflammatory agent is selected from ibuprofen, naproxen, fenoprofen, ketoprofen, flurbiprofen, suprofen, benoxaprofen, indoprofen, pirprofen, carprofen, loxoprofen, pranoprofen, alminoprofen, salicylic acid, diflunisal, salsalate, oxaprozin, indomethacin, sulindac, etodolac, ketorolac, diclofenac, piroxicam, meloxicam, tenoxican, lornoxicam, isoxicam, mefenamic acid, meclofenamic acid, flufenamic acid, tolfenamic acid, lumiracoxib and licofelone.

In certain embodiments, the NSAID is ibuprofen.

In certain embodiments, the NSAID is naproxen.

In certain embodiments, an anhydride polymer as described herein comprises one or more groups in the backbone that will provide morphine upon hydrolysis of the anhydride polymer.

In certain embodiments, the backbone comprises one or more groups of formula (V):

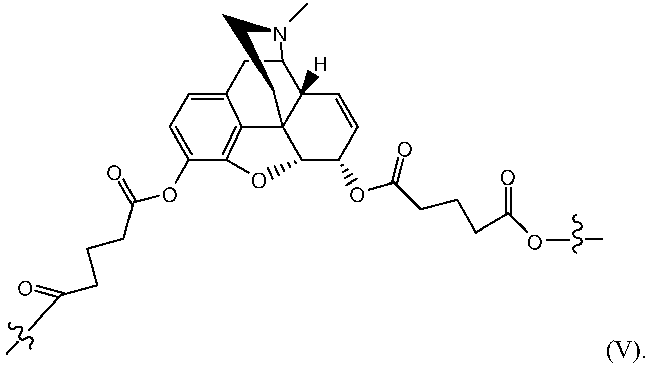

In certain embodiments, an anhydride polymer as described herein comprises 2-200 repeating groups of formula (V). In certain embodiments, an anhydride polymer as described herein comprises about 2-150 repeating groups of formula (V). In certain embodiments, an anhydride polymer as described herein comprises about 2-100 repeating groups of formula (V). In certain embodiments, an anhydride polymer as described herein comprises about 2-75 repeating groups of formula (V). In certain embodiments, an anhydride polymer as described herein comprises about 25-75 repeating groups of formula (V). In certain embodiments, an anhydride polymer as described herein comprises about 40-60 repeating groups of formula (V).

In certain embodiments, an anhydride polymer as described herein comprises at least 2, 3, 4, 5, 6, 7, 8, or 9 repeating groups of formula (V).

In certain embodiments, an anhydride polymer as described herein and prepared as described herein has an average molecular weight of about 10,000 daltons to about 60,000 daltons. In certain embodiments, the average molecular weight is at least about 10,000 daltons. In certain embodiments, the average molecular weight is at least about 15,000 daltons. In certain embodiments, the average molecular weight is at least about 20,000 daltons. In certain embodiments, the average molecular weight is at least about 25,000 daltons. In certain embodiments, the average molecular weight is at least about 30,000 daltons. In certain embodiments, the average molecular weight is at least about 35,000 daltons. In certain embodiments, the average molecular weight is at least about 40,000 daltons. In certain embodiments, the average molecular weight is at least about 45,000 daltons. In certain embodiments, the average molecular weight is at least about 50,000 daltons. In certain embodiments, the average molecular weight is at least about 55,000 daltons. In certain embodiments, the average molecular weight is at least about 60,000 daltons.

The anhydride polymers as described herein may be processed into microspheres using known methods and procedures commonly employed in the field of synthetic polymers, *e.g*., as described in the Examples. In certain embodiments, the diameter of the microsphere is between about 10 µm to about 100 µm. In certain embodiments, the diameter of the microsphere is between about 10 µm to about 90 µm. In certain embodiments, the diameter of the microsphere is between about 10 µm to about 80 µm. In certain embodiments, the diameter of the microsphere is between about 10 µm to about 70 µm. In certain embodiments, the diameter of the microsphere is between about 10 µm to about 60 µm. In certain embodiments, the diameter of the microsphere is between about 10 µm to about 50 µm. In certain embodiments, the diameter of the microsphere is between about 10 µm to about 40 µm. In certain embodiments, the diameter of the microsphere is between about 10 µm to about 30 µm. In certain embodiments, the diameter of the microsphere is about 30 µm. In certain embodiments, the diameter of the microsphere is about 20 µm. In certain embodiments, the diameter of the microsphere is about 10 µm.

Accordingly, certain embodiments of the present invention provide a microsphere that comprises an anhydride polymer as described herein.

Hence, certain embodiments of the present invention provide a microsphere that comprises an anhydride polymer which comprises a backbone that comprises one or more groups in the backbone that will provide morphine upon hydrolysis of the polymer and one or more pendant residues of a non-steroidal anti-inflammatory.

Certain embodiments of the present invention provide a pharmaceutical composition comprising an anhydride polymer or microsphere as described herein and a pharmaceutically acceptable carrier.

The present document describes a method to treat pain in a mammal, e.g., a human, comprising administering a first polymer comprising repeating units that form a biodegradable backbone, wherein morphine is incorporated into the backbone to the mammal.

The present document also describes a method to treat pain in a mammal, *e.g.,* a human, comprising administering a polymer as described herein to the mammal. In certain embodiments of the invention the polymer is formulated into microspheres.

The present document also describes a method to treat pain in a mammal, *e.g.,* a human, comprising administering a first polymer comprising a backbone which comprises one or more groups in the backbone that will provide morphine upon hydrolysis of the polymer, and a second polymer, as described herein, to the mammal.

Certain embodiments of the present invention provide an anhydride polymer as described herein for use in medical treatment.

In certain embodiments of the invention the first or second polymer is formulated into microspheres. In certain embodiments of the invention the first and second polymers are formulated into microspheres.

Certain embodiments of the present invention provide an anhydride polymer as described herein for use in treating pain.

The anhydride polymer as described herein may be administered locally.

The anhydride polymer as described herein may be administered by injection.

The present document describes processes and intermediates disclosed herein that are useful for preparing an anhydride polymer of the invention (*e.g*. the Examples). The intermediates described herein may have therapeutic activity, and therefore, may also be used for the treatment of pain.

The polymers and microspheres of the invention can be formulated as compositions, e.g., pharmaceutical compositions, and administered to a mammalian host, such as a human patient in a variety of forms adapted to the chosen route of administration, *e.g*., orally or parenterally, by intravenous, intramuscular, topical or subcutaneous routes.

Thus, the present polymers and microspheres may be systemically administered, *e.g*., orally, in combination with a pharmaceutically acceptable vehicle such as an inert diluent, excipient or an assimilable edible carrier. They may be enclosed in hard or soft shell gelatin capsules, may be compressed into tablets, or may be incorporated directly with the food of the patient's diet. For oral therapeutic administration, the polymers and microspheres may be combined with one or more excipients and used in the form of ingestible tablets, buccal tablets, troches, capsules, elixirs, suspensions, syrups, wafers, and the like. Such compositions and preparations should contain at least 0.1 % of the polymers or microspheres. The percentage of the compositions and preparations may, of course, be varied and may conveniently be between about 2 to about 90% of the weight of a given unit dosage form. The amount of the polymers or microspheres in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a poly(ethylene glycol). Various other materials may be present as coatings or to otherwise modify the physical form of the solid unit dosage form. For instance, tablets, pills, or capsules may be coated with gelatin, wax, shellac or sugar and the like. A syrup or elixir may contain the polymers or microspheres, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the polymers or microspheres may be incorporated into sustained-release preparations, particles, and devices.

The present anhydride polymers or microspheres may also be administered intravenously or intramuscularly by infusion or injection. Solutions of the polymer or microspheres can be prepared in water, optionally mixed with a nontoxic surfactant. Dispersions can also be prepared in glycerol, liquid poly(ethylene glycols), triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. The ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid poly(ethylene glycols), and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the present anhydride polymers or microspheres in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present anhydride polymers or microspheres may be applied in pure form, *e.g.,* when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina, nanoparticles, and the like. Useful liquid carriers include water, alcohols or glycols or water-alcohol/glycol blends, in which the anhydride polymers or microspheres can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the present anhydride polymers and microspheres to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the anhydride polymers or microspheres of the invention can be determined by comparing their *in vitro* activity, and *in vivo* activity in animal models. Methods for the extrapolation of effective dosages in mice, and other animals, to humans are known to the art; for example, see U.S. Pat. No. 4,938,949.

The amount of the anhydride polymers or microspheres of the invention, required for use in treatment will vary with the route of administration, the nature of the condition being treated and the age and condition of the patient and will be ultimately at the discretion of the attendant physician or clinician.

The anhydride polymers or microspheres of the invention can be conveniently formulated in unit dosage form. In one embodiment, the invention provides a composition comprising the anhydride polymers or microspheres of the invention formulated in such a unit dosage form.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations.

Anhydride polymers and microspheres of the invention can also be administered in combination with other therapeutic agents, for example, other agents that are useful for the treatment of pain, such as, *e.g*., NSAIDs or opiates. Examples of such agents include paracetamol, parecoxib, nefopam, tramadol, remifentanil, pethidine, ketamine, fentanyl, buprenorphine, lidocaine, dilofenac, rofecoxib, nalbuphine, celecoxib, etoricoxib, lumiracoxib, methadone, venlafaxine, imipramine, duloxetine, bupropion, gabapentin, pregabalin, lamotrigine, oxycodone HCl, alfentanil, sufentanil, diamorphine and butorphanol or agents listed in Drugs, 67(15), 2121-2133 (2007). Accordingly, one embodiment the invention also provides a composition comprising anhydride polymers and microspheres of the invention, at least one other therapeutic agent, and a pharmaceutically acceptable diluent or carrier. This document also discloses a kit comprising polymers and microspheres of the invention, at least one other therapeutic agent, packaging material, and instructions for administering the comprising anhydride polymers and microspheres of the invention and the other therapeutic agent or agents to an animal (e.g., human) to treat pain.

Certain embodiments of the invention will now be illustrated by the following non-limiting Examples.

### Example 1

Opiates are the drugs of choice for the treatment of moderate to severe acute and chronic pain.^{1,2} Morphine is the most important³ and widely used drug⁴ to control acute and chronic pain. Its short half-life in plasma of 1.7 to 4.5 hours,⁵ its analgesic effect that last 4 to 6 hours,⁵ and the tendency of people to develop of tolerance¹ to the drug leads frequent dosing (every 3 to 4 hours)^{1,5,6} and patient discomfort. Many efforts have been made to develop a controlled and sustained release formulation for morphine and other opiates.

Acrylic resins such as Eudragit® are widely used materials for controlled and sustained release of morphine. Morphine-Eudragit complexes prepared can control the release of morphine for up to 8 hours.^{6,7} Although paraffin tablets,⁵ poly(lactic-co-glycolic) microspheres,⁸ and ethyl cellulose microspheres⁹ were developed as controlled release systems for morphine, none demonstrate sustained release for more than one day. Only one publication reports the incorporation of morphine into a polymer backbone, a polyurethane, but is not biodegradable and did not sustain release for more than a day.¹⁰

Poly(anhydride ester)s (PAEs) are materials that biodegrade into non-toxic components and have been used for many years as polymer matrices (e.g. implants, films) and particulates (*e.g.* micro/nanoparticles) for drug delivery.^{11,12} Both the ester and anhydride moieties are susceptible to hydrolytic degradation.¹² Their biocompatibilities and susceptibility to degradation makes the PAEs useful biomaterials for controlled and sustained release of bioactive molecules.

In recent years, instead of physically admixing or encapsulating drugs within a polymer matrix or particulate, bioactive molecules containing two functional groups have been incorporated into PAE backbones.¹³⁻¹⁷ Chemical incorporation of bioactive molecules into the polymer backbone increases drug loading when compared to physical incorporation because the drug delivery system is mostly the drug itself.^{13,14} A polymeric version of a drug can be readily injected or ingested to reach the target site. The drug is then released *via* the hydrolytic degradation of the polymer backbone.

A PAE-based system (polyopiate) could help to overcome the limitations of the existing morphine controlled release systems by increasing the overall release time (i.e. sustained release). This polymer could be formulated into microspheres for localized drug release. It would also be advantageous in the prevention of abuse because the drug is not immediately available.

Additionally, the ability of an opiate-non steroidal anti-inflammatory (NSAID) drug combined treatment for acute and chronic pain has been reported.^{1,18-23} A combined treatment of oral or intravenous administration of an opiate and NSAID helps to reduce the amount of opiate needed^{18,21,22} and delays the development of tolerance.^{1,18} However, side effects associated with NSAIDs such as gastrointestinal discomfort, bleeding and ulceration increased.^{18,21-23}

A combined treatment with opiate-based PAE and NSAID-based PAE microspheres is described herein, which may be used to treat chronic and acute pain. This system will control and sustain the release of both drugs ultimately delaying the development of opiate resistance and reducing the side effects associated with the NSAIDs.

### 1.2. Controlled, sustained and localized release of a potent analgesic: PolyMorphine microspheres

Morphine has two reactive functional groups from which polymerization can take place. The polymer version of morphine will be synthesized, characterized and then formulated into microspheres.

### 1.3. Completing the combined treatment: Incorporation of propionic acid derived NSAIDs into PAE backbone and microspheres formation

Available synthetic methods were applied to incorporate molecules that have only one hydroxyl functional group as pendant groups into PAEs.²⁴ Antiseptics, antibacterial, food preservatives, etc. have been incorporated into PAEs using this method.

Propionic acid derived NSAIDs (**shown below**) will be incorporated into PAE backbones as pendant groups to take advantage of their medical properties and reduce the harmful side effects. These polymers will be formulated into microspheres and used in combination with PolyMorphine to treat acute and chronic pain. 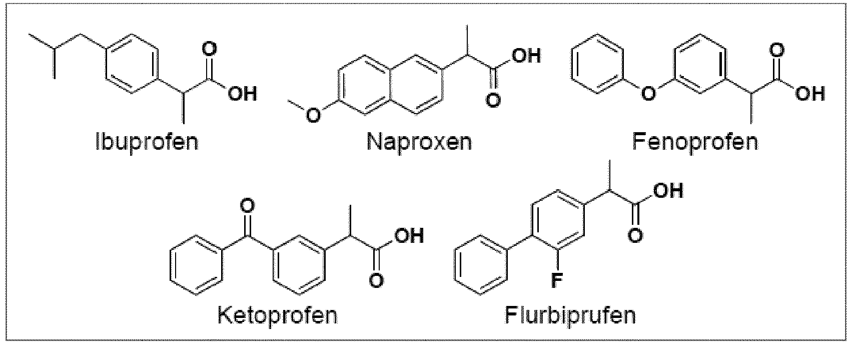 Structure of propionic acid derived NSAIDs.

### 2. Results

### 2.1. Salicylic acid release from polymer microspheres

Polymer microspheres are systems widely used as drug delivery devices. They have spherical shape and sizes varying from 1 to 1,000 µm in diameter.²⁵ In general, biodegradable microspheres are used for delivery of drug molecules,²⁵⁻²⁸ DNA,^{25,27} and proteins.^{25,29} When used as drug delivery devices, the drug may be encapsulated within a polymer matrix.²⁷ Its major benefits involve the lack of surgery required for implantation (i.e. can be injected in suspension) or removal (i.e. completely degrade over time).³⁰ Other benefits include controlled release of the drug and specificity of localized delivery.²⁵

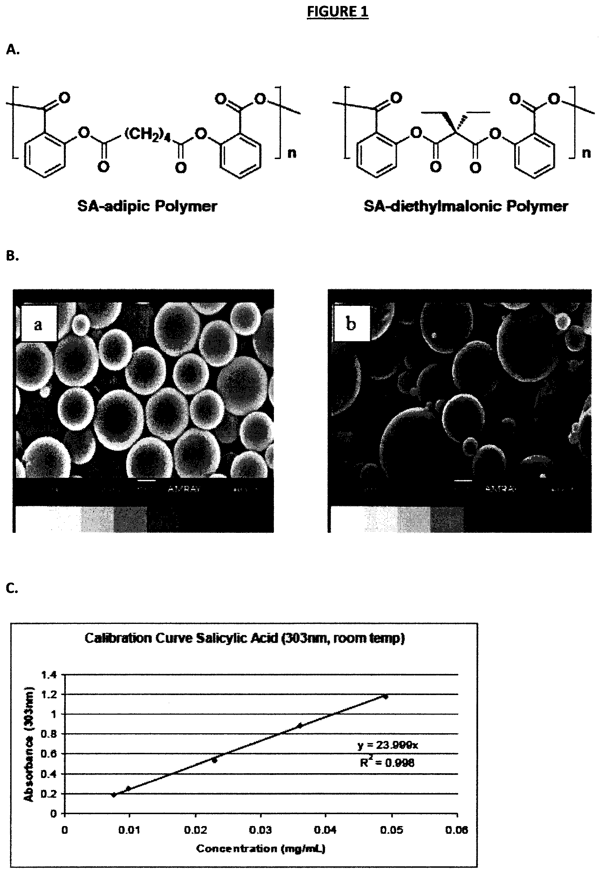

Salicylate-based PAEs were formulated into microspheres using a modification of a previously published oil-in-water single emulsion solvent evaporation technique³¹ obtaining yields of approximately 85%. Two polymers that incorporate salicylic acid (SA) into the polymer backbone were used: one with linear aliphatic linker (adipic) and the other with a branched aliphatic linker (diethylmalonic), the structures of these polymers are shown in **Figure 1A**. SA-diethylmalonic polymer was used to produce microspheres that sustain the release of SA for a longer period of time (e.g. months compared to weeks or days) due to the hydrophobicity of the linker. SA-adipic polymer was a control because it degrades within weeks.

In general, salicylate-based polymer (0.50 g) was dissolved in 3 mL of dichloromethane and slowly added to 80 mL 1% aqueous poly(vinyl alcohol) solution at room temperature. The emulsion was homogenized for 2 min at approximately 10,000 rpm using a homogenizer. The homogenized solution was then left stirring for 2 h to allow microsphere formation by solvent evaporation. Microspheres were washed twice with acidic water (pH 1) and isolated by centrifugation at 3,000 rpm for 10 min. Microspheres were frozen in a dry ice/acetone bath and lyophilized for 24 h.

The approximated size and the morphology of the microspheres were studied using scanning electron microscopy (SEM). Completely spherical microparticles of 10 to 30 µm in diameter were obtained as shown by SEM images on **Figure 1B****.**

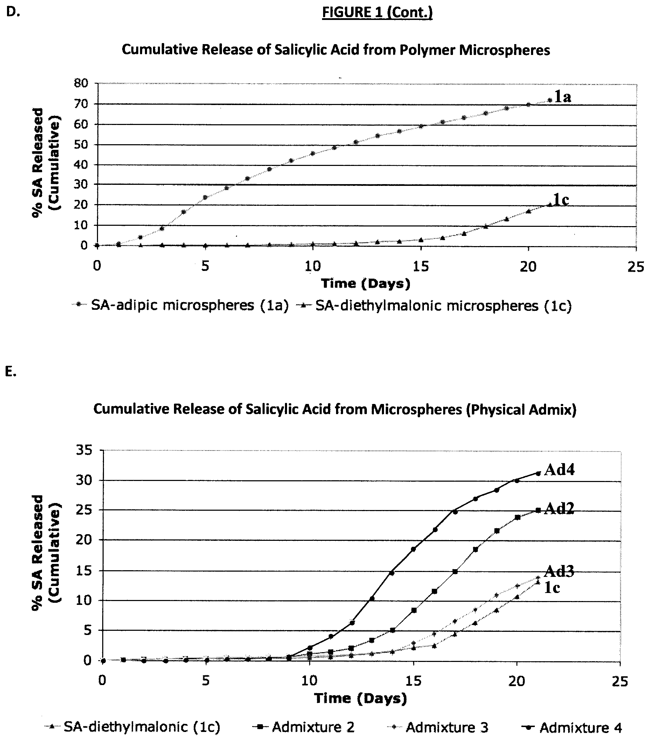

SA release from these microspheres was studied at 37°C in phosphate buffered saline (PBS) at pH 7.4 to mimic physiological conditions. The amount of SA released was monitored daily using Ultraviolet-visible (UV-vis) spectroscopy at 303 nm (wavelength at which SA absorbs and is not overlapped by the absorbance of the linkers). Data was taken in triplicate and calibrated against SA solutions of known concentrations (**Figure 1C**). **In** **Figure 1D** the release profile of the two types of polymer microspheres is shown.

Due to a lag time (i.e. period of time in which drug is not released) of approximately 10 days from the SA-diethylmalonic polymer microspheres, physical admixing of SA-containing compounds was used to obtain immediate release (**Table 1**). The physical admixtures shown in **Figure 1E** did not overcome the lag time; however, a 50:50 copolymer of SA-adipic:SA-diethylmalonic showed sustained drug release starting on day 2.

**Table 1. Physical admixtures used to prepare microspheres.**

| | Polymer | Admixture | % Admixture used |
|---|---|---|---|
| 2 | SA-diethylmalonic | SA | 10 |
| 3 | SA-diethylmalonic | Diacid SA-diethylmalonic | 10 |
| 4 | SA-diethylmalonic | SA-adipic polymer | 10 |

### 2.2. Salicylate-based PAE radiation exposure

It is necessary for drug delivery systems to meet the requirements of sterility when used *in vivo*.^{32,33} It is well known that most sterilization techniques such as sterilization by steam or dry heat, cannot be used for biodegradable polymers since they alter their properties.^{32,34} Exposure to gamma rays (γ-rays) and electron beams (e-beam) are the most commonly used to sterilize a large number of polymeric materials.³⁵

The Cobalt-60 (⁶⁰Co) γ-ray radiation sterilization is a simple, rapid, and effective process as it provides manufacturing benefit of prepackaging before sterilization.³⁵ It is successfully employed for the sterilization of thermoliable medical devices³⁴ such as poly(lactic-*co*-glycolic)-based drug delivery systems.³²⁻³⁴ In contrast, e-beam has considerably less penetrating ability, making this method inappropriate for thick or dense products.³⁵

Salicylate-based polymer with adipic linker **(****Figure 1A****, left)** was exposed to e-beam using a 5 MeV electron beam unit and γ-rays using ⁶⁰Co gamma cells. Polymer was exposed to radiation at 25 and 50 kGrays (kGy) ± 10 % by each method (being 25 kGy the most commonly validated dose used³⁴). An unexposed sample (0 kGy) was used as a control. Sterile Process Technology, a company that provides sterile processing to Johnson & Johnson operating companies in terminal sterilization and aseptic processing, performed the radiation exposure.

The polymer was chosen because it was extensively studied over the last few years and many of its properties are well known. The polymer was fully characterized before exposure and its properties were studied after radiation exposure **(Table 2** and **Table 3**).

**Table 2. Characterization data of SA-adipic PAE before and after γ-ray radiation exposure.**

| Sample | Before exposure | OkGy (traveler) | 25kGy | 50kGy |
|---|---|---|---|---|
| M_{w} (Da) (PDI) | 16,800 (1.8) | 15,300 (2.0) | 15,700 (2.1) | 14,700 (2.1) |
| T_{g} (°C) | 40.0 | 35.5 | 36.5 | 36.5 |
| T_{d}(°C) | 274.0 | 271.0 | 268.5 | 273.0 |
| H-NMR | ^{a}OK | ^{a}OK | ^{a}OK | ^{a}OK |
| IR (cm⁻¹) | 1791 (C=O anhydride) | 1791 (C=O anhydride) | 1792 (C=O anhydride) | 1791 (C=O anhydride) |
| | 1762 (C=O ester) | 1762 (C=O ester) | 1761 (C=O ester) | 1762 (C=O ester) |

| | | | | |
|---|---|---|---|---|
| ^{a}All ¹H-NMR chemical shifts were as expected. | | | | |

**Table 3. Characterization data of SA-adipic PAE before and after e-beam radiation exposure.**

| Samples | Before exposure | 0kGy (traveler) | 25kGy | 50kGy |
|---|---|---|---|---|
| M_{w} (Da) (PDI) | 16,800 (1.8) | 16,200 (1.7) | 15,800 (1.9) | 14,800 (2.9) |
| T_{g}(°C) | 40.0 | 36.0 | 36.5 | 36.0 |
| T_{d}(°C) | 274.0 | 272.5 | 273.0 | 272.0 |
| ¹H-NMR | ^{a}OK | ^{a}OK | ^{a}OK | ^{a}OK |
| IR | 1791 (C=O anhydride) | 1791 (C=O anhydride) | 1791 (C=O anhydride) | 1791 (C=O anhydride) |
| | 1762 (C=O ester) | 1762 (C=O ester) | 1762 (C=O ester) | 1761 (C=O ester) |

| | | | | |
|---|---|---|---|---|
| ^{a}All ¹H-NMR chemicals shifts were as expected. | | | | |

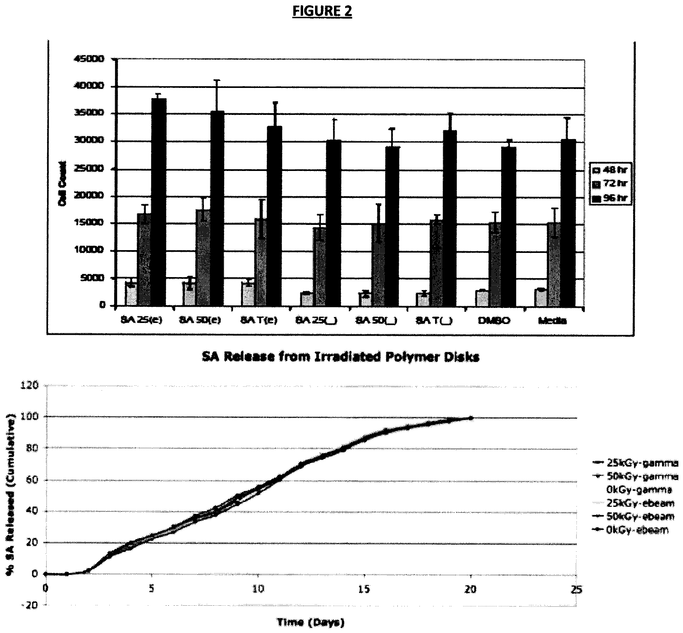

Neither e-beam nor γ-rays significantly affected the properties of the polymer compared to the traveler control (exposed to 0 kGy). The molecular weight (M_{w}), decomposition temperature (T_{d}), glass transition temperature (Tg), proton nuclear magnetic resonance (¹H-NMR) spectrum, infrared (IR) spectrum, cell compatibility (evaluated by culturing NCTC clone 929, strain L, mouse areolar fibroblast cells in media containing the dissolved polymer), and drug release profile did not change significantly. **Figure 2** shows the cell compatibility data as well as the drug release profile of the polymer after radiation exposure.

### 2.3. PolyOpiates

Nalbuphine, an agonist-antagonist used as an analgesic,³⁷ was chosen as a model compound for developing the synthesis of the polymer precursor (i.e. diacid) and polymeric version of morphine. The structural similarities to morphine as shown below make nalbuphine a good prototype for developing the synthetic method that will be used to polymerize morphine. It is important to develop this synthetic method with a molecule similar to morphine, as the supply of morphine is limited because it is an expensive regulated drug.

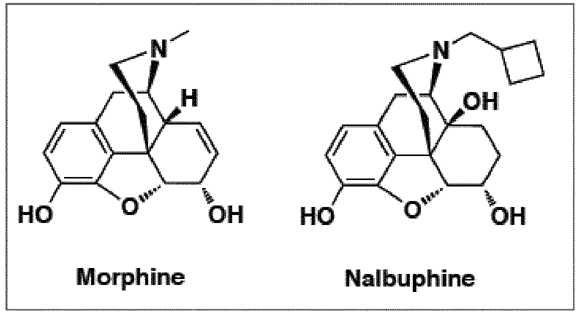

The nalbuphine-based polymer not only helps to develop a synthetic method and work-up method for the polymerization of morphine, but it can also be used as a biodegradable polymer for drug delivery. The analgesic properties of nalbuphine can be utilized for localized, control, and sustain drug release when incorporated into a PAE. The polymerization of nalbuphine will help reduce the number of times the drug is administrated and prolong its analgesic effect.

The nalbuphine-based diacid was synthesized by reacting neutral nalbuphine with glutaric anhydride in a ring-opening reaction catalyzed by a base **(Scheme 1a).**

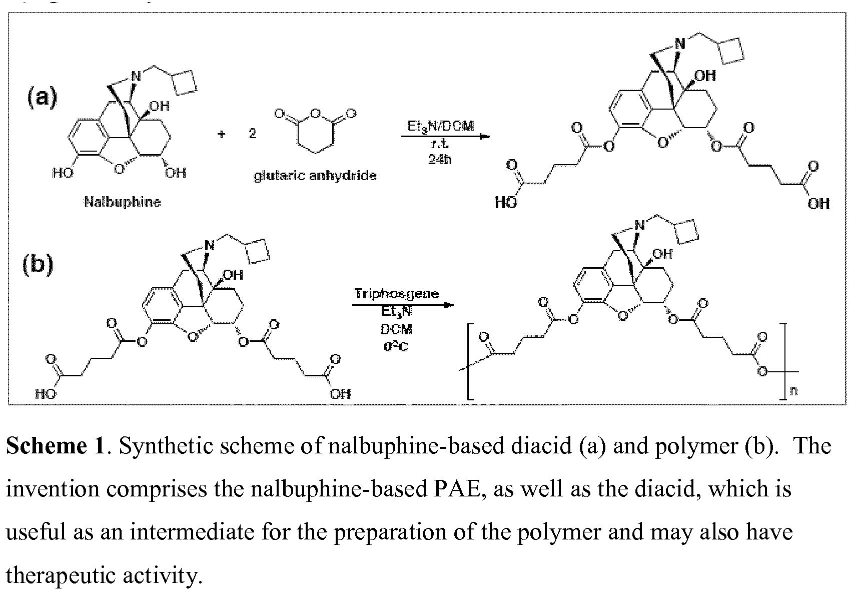

The isolation of the diacid was challenging; the standard procedures used in previously published methods¹³⁻¹⁶ (which involves the precipitation of the product from acidic water) did not work. After attempting several methods, the product was obtained by drying the reaction mixture under high vacuum, dissolving the yellow gel obtained in a minimal amount of ethyl acetate and precipitating the solid over an excess of hexanes.

Nalbuphine-based diacid was obtained as a white foam (**Table 4** shows data for the characterization of the diacid). **Table 5** shows data for the characterization of the nalbuphine-based PAE. Mass spectrometry (MS) was used to determine the M_{w}, FT-IR was used to confirm the formation of the ester bond in the diacid, the T_{d} was determined using thermogravimetric analysis (TGA) and the melting point (Tₘ) using differential scanning calorimetry (DSC). Note that ¹HNMR was not used because of the complexity of the spectrum and the overlap of the signals.

**Table 4. Characterization data of nalbuphine-based diacid.**

| Property | Polymer Precursor |
|---|---|
| M_{w} | 585.65 |
| Tₘ | 97°C |
| T_{d} | 215 °C, |
| IR | 1773 cm⁻¹ (C=O carboxylic acid), 1693 cm⁻¹ (C=O ester) |

**Table 5. Characterization data of nalbuphine-based PAE.**

| Property | Polymer |
|---|---|
| M_{w} | 20,000 Da |
| T_{d} | 200 °C |
| T_{g} | 122 °C |
| IR | 1859, 1735 cm⁻¹ (C=O anhydride), 1759 cm⁻¹ (C=O ester) |

### 3. Combined Opiate-NSAID Treatment Research

Described herein is the development of a combined opiate-NSAID treatment for acute and chronic pain that can control and sustain the release of both drugs (i.e. morphine and NSAID), reduce the side effects associated with the NSAIDs (i.e. a high concentration of drugs would not be available in the blood to allow absorption by the gastrointestinal tract), reduce the amount of opiate needed, and delay the development of opiate resistance.

### 3.1. Controlled, sustained and localized release of a potent analgesic: PolyMorphine microspheres

### 3.1.1. PolyMorphine synthesis

Morphine will be incorporated into a PAE backbone to control and sustain its release. The rationale behind the synthesis of this polymer is: 1) to increase specificity through localized drug release, 2) to eliminate the requirements for frequent dosing by prolonging the release, 3) prevent drug abuse since the drug is not immediately available, and/or 4) to reduce the occurrence of undesired side-effects. PolyMorphine synthesis will be performed as described in **Section 2.3** and shown in **Scheme 2** (below).

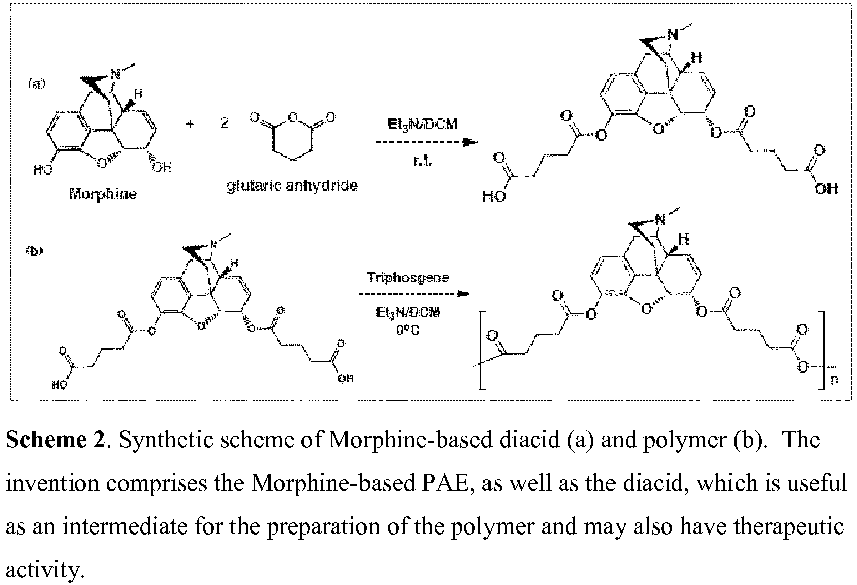

### 3.1.2. Characterization

**MS**: The M_{w} of the diacid will be determined using this technique.

**Elemental Analysis**: The structure of the diacid and its purity will be determined in part using this technique.

**GPC**: The M_{w} of the polymer will be determined using this method with respect to polystyrene standards in dichloromethane.

**IR**: The formation of the ester bonds and carboxylic acids in the diacid and the presence of the ester and anhydride bonds in the polymer will be determined with this method solvent casting the samples on NaCl salt plates.

**TGA**: The T_{d} will be determined to ensure the material's stability after storage and temperature exposure.

**DCS**: Both the Tₘ of the diacid and the T_{g} of the polymer will be determined using DSC. Note: ¹H-NMR will not be used due to complexity and overlapping of signals.

### 3.1.3. Microspheres preparation

PolyMorphine will be formulated into microspheres for better localization of the drug release when applied *in vivo.* Microspheres will be prepared using the oil-in-water single emulsion solvent evaporation method described in **Section 2**.**1**.

**SEM**: The morphology and approximate size of the microspheres will be determined using this type of microscopy. The samples will be coated with Au/Pd and then analyzed under the microscope.

### 3.1.4. Microspheres sterilization

γ**-Ray Radiation**: PolyMorphine microspheres will be exposed to γ-ray radiation at 25 kGy using ⁶⁰Co cells at the Sterile Process Technology facilities. This technique is chosen because it is supported by the results discussed in **Section 2**.**2** and by the literature.³¹⁻³³

### 3.1.5. In vitro drug release from microspheres

*In vitro* **release study**: PolyMorphine microspheres will be suspended in PBS at pH 7.4 and incubated at 37°C. Every day, microspheres will be centrifuged down and an aliquot of PBS taken and analyzed to determine the amount of free drug and diacid released. The aliquot will be replaced by the same amount off fresh PBS and the microspheres resuspended.

The drug release profile may be modified by altering the linker, synthesizing co-polymers and/or using physical admixtures.

**High Performance Liquid Chromatography** (HPLC): An HPLC method will be developed to quantify the amount of morphine released at a specific time point (daily release). Previously published methods for the detection of morphine by HPLC^{6,8} will be tested to find the most suitable one.

### 3.1.6. Anticipated results

Based upon the structural similarities between nalbuphine and morphine, the same synthetic methods should be able to be used to yield the product polymer.

### 3.2. Completing the combined treatment: incorporation of propionic acid derived NSAIDs into PAE backbone and microspheres

### 3.2.1. Propionic acid derivate NSAIDs polymerization

Propionic acid derivatives NSAIDs have gained approval for use as over the counter analgesics to treat pain, fever, and inflammation. This new series of compounds could potentially serve as a replacement for aspirin.³⁸ The major disadvantage of propionic acid derived NSAIDs is their tendency to induce gastric and intestinal erosion, bleeding and ulceration.³⁸⁻⁴³ Multiple efforts have been made to minimize gastric effects by masking the carboxylic acid groups. This is achieved by using a non-polymeric³⁹ or a polymeric prodrug with the NSAIDs as pendant groups.⁴⁰⁻⁴⁶

Drug loading achieved by the polymeric prodrug is up to 30-40% and the release up to approximately 70 % in 24 h.⁴⁵ These polymeric prodrugs do not load high amounts of drug and do not sustain the release for more than one day. Even though they are made of biocompatible polymers, they are made from nonbiodegradable polymers such as methacrylic polymers,^{40,43,45} vinyl ether polymers,⁴¹ and polyoxyethelene.⁴⁶

Considering these drugs do not have two reactive functional groups or one highly reactive functional group that can be used to prepare a biodegradable polymer using previously published methods,^{13-17,24} it is important to incorporate other synthetic methods to polymerize these less reactive molecules. In order to chemically incorporate propionic acid derived NSAIDs (*see* compounds shown in **Section 1.3**) into PAEs, a different methodology to prepare the polymer precursor has to be applied.

For *in vivo* applications, choosing a biocompatible "linker" is important. It must be biocompatible in order to reduce side effects and/or toxicity after hydrolytic degradation. Naturally occurring acids are a good choice because they are non-toxic and biocompatible. Tartaric acid is a good candidate because it occurs naturally in fruits and is used as an antioxidant.⁴⁷ Using tartaric acid as a "linker" molecule would be beneficial because it is biocompatible and can impart its antioxidant properties (i.e. removing potentially damaging oxidizing agents).

To synthesize the polymer precursor, dicyclohexylcarbodiimide (DCC) coupling could be used. The carboxyl groups of tartaric acid must be protected before DCC coupling to avoid random branching by reaction of carboxyl and hydroxyl groups of tartaric acid competing with the reaction of in hydroxyl groups of tartaric acid and carboxyl groups of the bioactive molecule (**Scheme 3a**). 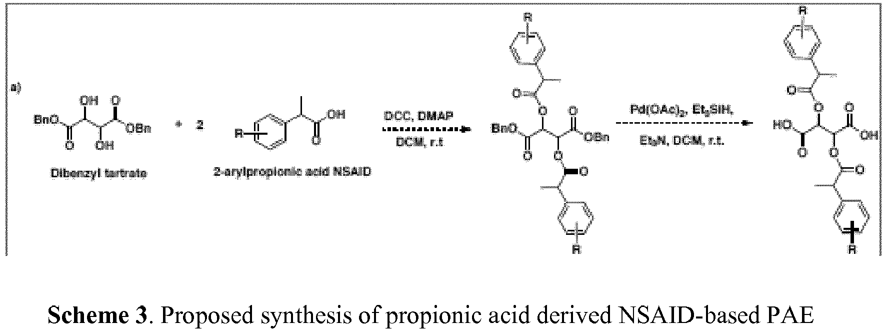 diacid (a). The invention comprises the diacid, which is useful as an intermediate for the preparation of the polymer and may also have therapeutic activity.

Benzyl-protected tartaric acid (i.e. dibenzyl tartrate) is commercially available and can be used as received to perform the reaction. The deprotection step used in used by Lamidey et al. to synthesize chicoric acid⁴⁸ (**Scheme 4**) will be applied for the "diacid" synthesis. The polymers can be prepared as discussed in Example 2.

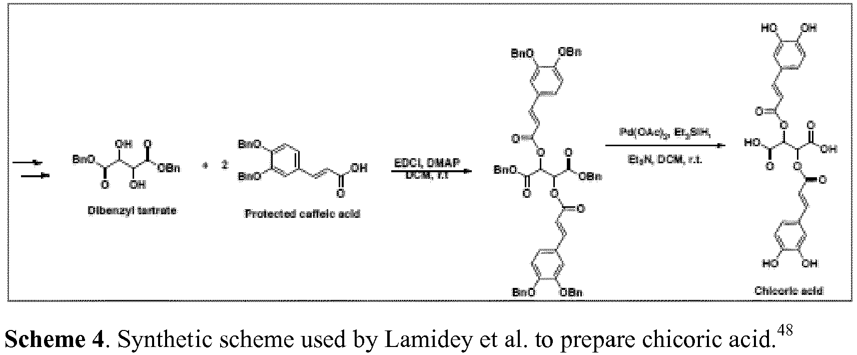

### 3.2.2. Characterization

The characterization of both "diacid" and polymer will be performed using the methods described in **Section 3.1.2** in addition to ¹H-NMR.

### 3.2.3. Microspheres preparation See Section 3.1.3.

### 3.2.4. Microspheres sterilization See Section 3.1.4.

### 3.2.5. In vitro release from microspheres See Section 3.1.5.

**HPLC**: UV/vis spectroscopy is widely used to quantify the amount of drugs released from drug delivery systems, however, having two bioactive molecules in the polymer make independent detection of each component difficult by this method. An HPLC method must be developed to quantify the amount of bioactive released at a specific time point. Previously published methods for the detection of these bioactive molecules by HPLC^{39,40} will be tested to find the most suitable one.

### 3.2.6. Anticipated results

A possible limitation may be obtaining overall low yields. However, the reaction conditions and methods can be optimized to improve the yields.

### 3.3 In vivo studies

Using rodent nociceptive assays to measure the ability of opiate-based PAE and NSAID-based PAE microspheres to produce analgesia, microspheres properties will be determined using four different studies. First, the dosing of the polymer microspheres for their effective analgesia relative to a standard dose-response curve of acutely administrated morphine/NSAID will be titrated. Second, the sustained analgesia time course will be determined. Third, the extent of tolerance development as compared with the standard procedure of chronic morphine administration will be assessed. Fourth, the extent of drug preference compared with morphine itself will be measured.

### 4. Summary

A combined treatment with morphine-based PAE and NSAID-based PAE microspheres may be used to treat chronic and acute pain, reduce undesired side effects of NSAIDs, control and sustain the release of both drugs, and delay the development of morphine resistance.

### 5. References

1) Koppert, W. Acute Pain 2007, 9, 21.
2) Hagen, N.A.; Thrilwell, M.; Eisenhoffer, J.; Quigley, P.; Harsanyi, Z.; Darke, A. J. Pain Symptom. Manage. 2005, 29, 80.
3) Vermeire, A.; Remon, J.P.; Int. J. Pharm. 1999, 187, 17.
4) Abou hammoud, H.; Simon, N.; Urien, S.; Rion, B.; Lechat, P.; Aubrun, F. Pain 2009, 144, 139.
5) Olsson, B.; Wagner, Z.G.; Månsson, P.; RGNARSSON, G. Int. J. Pharm. 1995, 119,223.
6) Holgado, M.A.; Iruin, A.; Alvarez-Fuentes, J.; Fernández-Arévalo, M. Eur. J. Pharm. Biopharm. 2008, 70, 544.
7) Alvarez-Fuentes, J.; Fernández-Arévalo, M.; Holgado, M.A.; Caraballo, I.; Rabasco, A.M.; Micó J.A.; Rojas, O.; Ortega-Alvaro, A. Int. J. Pharm. 1996, 139, 237.
8) Polard, E.; Le Corre, P.; Chevanne, F.; Le Verge, R. Int. J. Pharm. 1996, 134, 37.
9) Morales, M.E.; Gallardo Lara, V.; Calpena, A.C.; Domenech, J.; Ruiz, M.A. J. Control. Release. 2004, 95, 75.
10) Mahkam, M.; Sharif-Sanjani, N. Polym. Deg. Stab. 2003, 80, 199.
11) Jain, J.P.; Modi, S.; Domb, A.J.; Kumar, N. J. Control. Release 2005, 103, 541.
12) Edlund, U.; Albertsson, A.-C.; Adv. Drug Del. Rev. 2003, 55, 585.
13) Erdmann, L.; Uhrich, K.E. Biomaterials 2000, 21, 1941.
14) Anasrasiou, T.J.; Uhrich, K.E. J. Polym. Sci. 2003, 41, 3667.
15) Schmeltzer, R.C.; Schmalenberg, K.E.; Uhrich, K.E. Biomacromol. 2005, 6, 359.
16) Prudencio, A.; Schmeltzer, R.C.; Uhrich, K.E. Macromol. 2005, 38, 6895.
17) Schmeltzer, R.C.; Uhrich, K.E. Polym. Bull. 2006, 57, 281.
18) Mercadante, S.; Fulfaro, F.; Casuccio, A. Eur. J. Cancer 2002, 38, 1358.
19) Palnas, E.; Poveda, R.; Sánchez, S.; Romeo, A.; Puig, M.M. Eur. J. Pharm. 2003,482,223.
20) Kolesnikov, Y.; Sõritsa, D. Eur. J. Pharm. 2008, 569, 126.
21) Shaprio, A.; Zohar, E.; Hoppenstein, D.; Ifrach, N.; Jedeikin, R.; Fredman, B. J. Clin. Anesth. 2003, 15, 345.
22) Curatolo, M.; Sveticic, G. Best Pract. Res. Clin. Anaesthesiol. 2002, 16, 507.
23) Joshi, G.P. Ambul. Surg. 1999, 7, 12.
24) Prudencio, A.; Carbone, A.C.; Griffin, J.; Uhrich, K.E. Macromol. Rapid Commun. 2009,30, 1101.
25) Vasir, J.K.; Tambwekar, K.; Garg, S. Int. J. Pharm. 2003, 255, 13.
26) Freiberg, S.; Zhu, X.X. Int. J. Pharm. 2004, 282, 1.
27) Freitas, S.; Merkle, H.P.; Gander, B. J. Control. Rel. 2005, 102, 313.
28) Lee, K.Y.; Yuk, S.H. Prog. Polym. Sci. 2007, 32, 669.
29) Edlund, U.; Albertsson, A.-C. Adv. Polym. Sci. 2002, 157, 67.
30) Kipper, M.J.; Shen, E.; Determan, A.; Narasimhan, B. Biomaterials 2002, 23, 4405.
31) Yeagy, B.A.; Prudencio, A.; Schmeltzer, R.C.; Uhrich, K.E.; Cook, T.J. J. Microencap. 2006, 23, 643.
32) Williams, H.E.; Huxley, J.; Claybourn, M.; Booth, J.; Hobbs, M.; Meehan, E.; Clark, B. Polym. Deg. Stab. 2006, 91, 2171.
33) Montanari, L.; Costantini, M.' Signoretti, E.C.; Valvo, L.; Santucci, M.; Bartolomei, M.; Conti, B.; Genta, I. J. Control. Rel. 1998, 56, 219.
34) Martinez-Sancho, C.; Herrero-Vanrell, R.; Negro, S. J. Control. Rel. 2004, 99, 41.
35) Benson, R.S. Nucl. Instr. Meth. Phys. Res. 2002, 191, 752.
37) Schmidt, W.K.; Tam, S.W; Shotzberger, G.S.; Smith, D.H. Jr.; Clark, R.; Vernier, V.G. Drug Alcohol Depend. 1985, 14, 339.
38) Hersh, E.V.; Moore, P.A.; Ross, G.L. Clin Therap. 2000, 22, 500.
39) Khan, M.S.Y.; Akhter, M. Eur. J. Med. Chem. 2005, 40, 371.
40) Gallardo, A.; Parejo, C.; San Román, J. J. Control. Rel. 2001, 71,127.
41) Babazadeh, M. Int. J. Pharm. 2008, 356, 167.
42) Babazadeh, M. J. Appl. Polym. Sci. 2007, 104, 2403.
43) Wang, L.F.; Chiang, H.N.; Chen, W.B. J. Pharm. Pharmacol. 2002, 54, 1129.
44) Babazadeh, M. Int. J. Pharm. 2006, 316, 68.
45) Davaran, S.; Entezami, A.A. Eur. Polym. J. 1998, 34, 187.
46) Bonina, F.P.; Puglia, C.; Barbuzzi, T.; de Capariis, P.; Palagiano, F.; Rimoli, M.G.; Saija, A. Eur.J. Pharm. Sci. 2001, 14, 123.
47) DeBolt, S.; Cook, D.R.; Ford, C.M. Proc. Natl. Acad. Sci. USA 2006, 103, 5608.
48) Lamidey, A.-M.; Fernon, L.; Pouységun, L.; Delattre, C.; Quideau, S. Helv. Chim. Acta 2002, 85, 2328.

### Example 2

Drugs administered by conventional routes (i.e., enteral and parenteral) are distributed throughout the body to target and non-target sites. This can result in increased side effects and, if the drug has a relatively short half-life, frequent dosing will be required to maintain drug levels within therapeutic levels. For example, ibuprofen (1) and naproxen (2), shown below, are non-steroidal anti-inflammatory drugs (NSAIDs) with relatively short half-life in plasma (2.1 and 14 hours, respectively) (Brooks, New England Journal of Medicine 1991, 324 (24), 1716-1725). When administered by conventional routes, severe gastrointestinal (GI) side effects occur such as stomach ulceration, bleeding, and perforation. 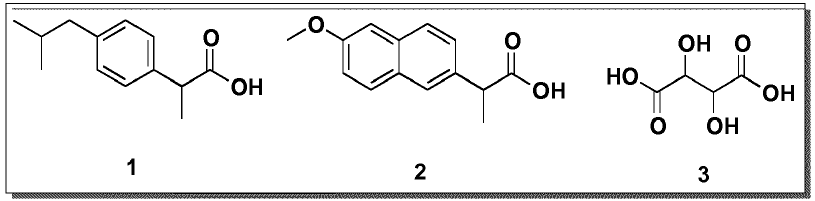 Chemical structures of ibuprofen (1), naproxen (2), and tartaric acid (3).

Drug delivery systems have been developed to localize drug release, thereby decreasing the side effects associated with systemic drug administration and prolonging the duration of the drug effect. The preparation of microparticles conjugating or encapsulating **1** has been studied (Arica, et al., Journal of Microencapsulation 2005, 22 (2), 153-165; Thompson, et al., Journal of Microencapsulation 2009, 26 (8), 676-683; Fernández-Carballido, International Journal of Pharmaceutics 2004, 279, 33-41). The major issues associated with these types of drug delivery systems are the low drug loading and burst release of the drug. Acrylate polymers have been widely studied for the chemical incorporation of 1 into the polymer backbone (Khan, et al., European Journal of Medicinal Chemistry 2005, 40 (4), 371-376). Although acrylic polymers are biocompatible, they are not biodegradable. Therefore when the entire drug is released, the polymer could remain in the body which could cause patient discomfort. Despite the limitations, these drug delivery systems have been shown to lower the side effects associated with the drug and to increase the duration of the drug effects.

The chemical incorporation of bioactive molecules into biodegradable polyanhydride backbones has been studied as a novel drug delivery method. For example, salicylic acid was chemically incorporated into a poly(anhydride-ester) (PAE) backbone achieving up to -75 wt.% drug loading, and the drug is released via hydrolytic degradation of the polymer (Schmeltzer, et al., Polymer Bulletin 2003, 49 (6), 441-448; Erdmann, et al., Biomaterials 2000, 21 (19), 1941-1946). Other examples are the chemical incorporation of phenolic derivatives (antiseptics) as pendant groups via ester bonds, achieving 48-58 wt.% loading (Prudencio, et al., Macromolecular Rapid Communications 2009, 30 (13), 1101-1108). The incorporation of **1** and **2** into PAE backbones is done as pendant groups and tartaric acid (**3**) can be used as backbone for this polymer. Tartaric acid is a naturally occurring and biocompatible compound that has antioxidant properties (DeBolt, et al., Proceedings of the National Academy of Sciences 2006, 103 (14), 5608-5613). Upon hydrolytic degradation, the bioactive molecules (**1** or **2**) having analgesic, antipyretic, and anti-inflammatory activities will be released with **3**, imparting its antioxidant properties.

The synthesis of the ibuprofen- and naproxen-based protected diacids (**5a** and **5b**, respectively) and their associated diacids (**6a** and **6b**, respectively) are described below (*see* Scheme 5). The synthesized polymer precursors will subsequently be used to prepare the respective polymers. Further work will include the synthesis, characterization, and the *in vitro and in vivo* testing of the polymers.

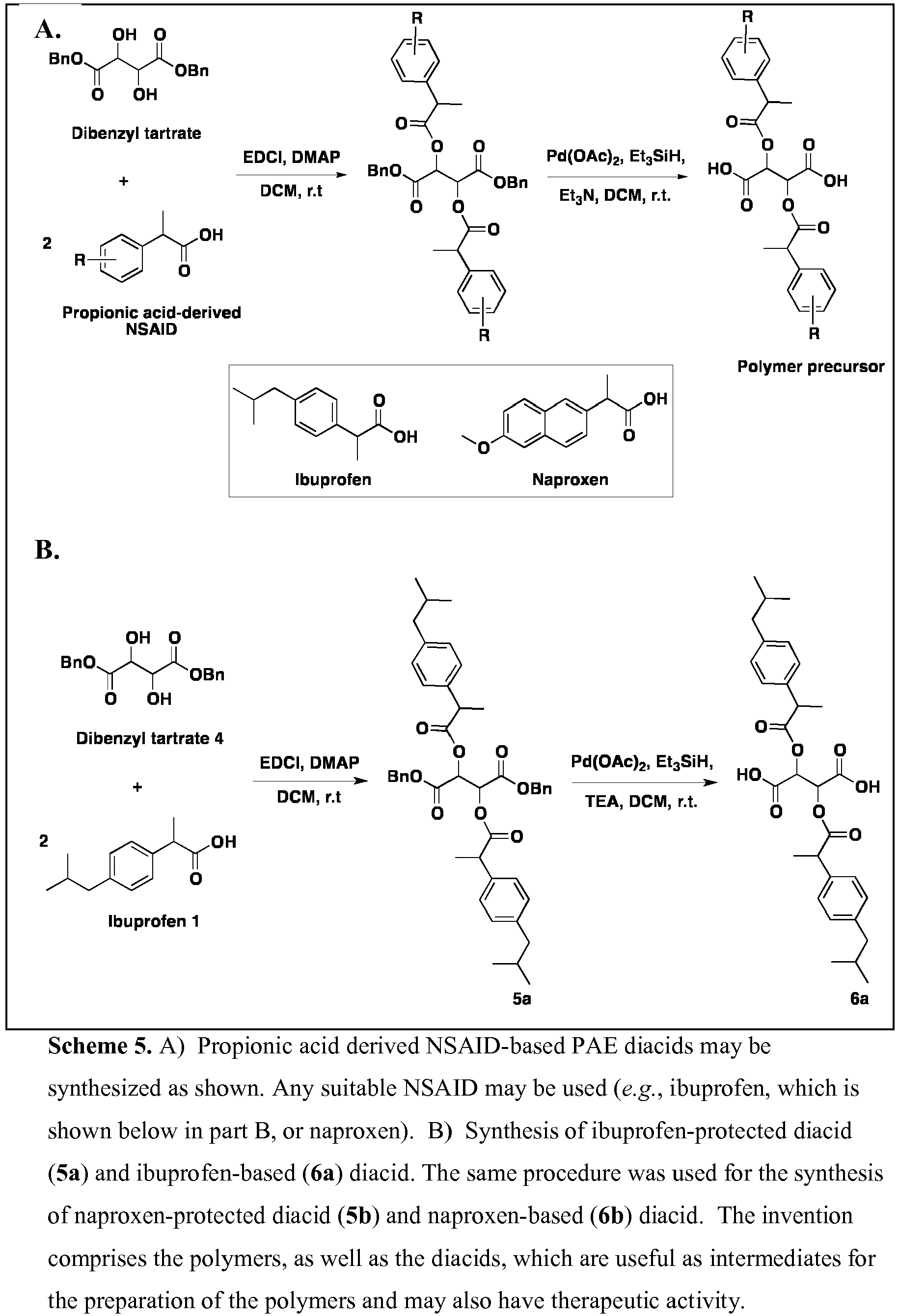

### MATERIAL AND METHODS

**Materials.** Naproxen and 1-[-3-(dimethylamino)propyl]-3-ethylcarbodiimide hydrochloride (EDCI) were purchased from Fisher Scientific (Pittsburg, PA). Unless otherwise specified, all other chemicals and reagents were purchased from Sigma-Aldrich (Milwaukee, WI) and used as received.

**Synthesis of 5a.** Ibuprofen (**1**, 3.21 g, 2.2 eq) was dissolved in anhydrous dichloromethane (DCM) and stirred under argon. Then 4-(dimethylamino)pyridine (DMAP, 1.90 g, 2.2 eq) was added. Dibenzyl-L-tartrate (**4**, 2.34 g, 1 eq) was dissolved in anhydrous DCM and added to the reaction mixture. It was followed by the addition of EDCI (5.96 g, 4.4 eq). The resulting yellowish solution was left stirring for 2 h. The reaction mixture was diluted with EtOAc and extracted with 10% KHSO₄ and saturated NaHCO₃. The organic layer was dried over MgSO₄ and solvent evaporated under reduced pressure to give a brown viscous oil that was dried *in vacuo* at room temperature overnight. Yield: 93 %. ¹H-NMR (CDCl₃, 500MHz): 7.30 (6H, m, ArH), 7.15 (6H, m, ArH), 7.05 (6H, m, ArH), 5.70 (2H, split, CH), 5.05-4.35 (3H, split, CH2), 3.80-3.60 (2H, dm, CH), 2.40 (4H, m, CH₂), 1.80 (2H, m, CH), 1.45 (6H, t, CH₃), 0.95 (12H, d, CH₃). ¹³C-NMR (CDCl₃, 500MHz): 173.5, 173.2, 165.7, 165.3, 140.9, 136.8, 134.8, 129.5, 128.6, 127.7, 71.1, 67.7, 45.2, 44.7, 30.4, 22.6, 18.3. IR: 1769 cm⁻¹ (C=O ester) and 1751 cm⁻¹ (C=O ester). MS: 724 [M + Na]. T_{d} = 237 °C.

**Synthesis of 5b.** Synthesis was performed using the procedure described for **5a**. Yield: **81** % (green foam). ¹H-NMR (CDCl₃, 500MHz): 7.60 (6H, t, ArH), 7.37 (2H, d, ArH), 7.18 (6H, m, ArH), 7.10 (2H, d, ArH), 7.03 (2H, d, ArH), 6.80 (3H, d, ArH), 5.61 (2H, s, CH), 4.25-4.15 (4H, d, CH₂), 3.94 (2H, m, CH), 3.87 (6H, s, OCH₃), 1.49 (6H, d, CH₃). ¹³C-NMR (CDCl₃, 500MHz): 173.5, 165.4, 158.0, 135.1, 134.6, 134.0, 129.6, 1291, 128.6, 128.5, 128.1, 128.0, 127.4, 126.5, 126.4, 119.3, 105.8, 71.2, 67.6, 55.5, 45.0, 18.4. IR: 1767 cm⁻¹ (C=O ester) and 1748 cm⁻¹ (C=O ester). MS: 777.2 [M + Na]. T_{d} = 294 °C.

**Synthesis of 6a.** To palladium (II) acetate [Pd(OAc)₂, 4.23 g, 2.5 eq], anhydrous DCM and triethylamine (TEA, 2.96 mL, 2.5 eq) were added under argon. Ibuprofen-based protected diacid (**5a**, 6.00 g, 1 eq) was dissolved in DCM and added dropwise to the reaction mixture. The solution was left stirring for 5 min and triethylsilane (Et₃SiH, 34.00 mL, 25 eq) was added dropwise via syringe pump. Reaction was left stirring at room temperature under argon overnight. MeOH was added and the mixture was filtered over celite to remove Pd. The filtrate was concentrated under reduced pressure and the orange residue obtained was diluted in EtOAc. The precipitate formed was removed via vacuum filtration. The filtrate was concentrated under reduced pressure, the orange liquid obtained was diluted in acetonitrile and extracted with hexanes. The acetonitrile layer was dried under reduced pressure. The orange residue obtained was diluted in EtOAc and extracted with water. The organic layer was dried over MgSO₄ and the solvent evaporated to give an orange, viscous oil. Yield: 77 %. ¹H-NMR (CDCl₃, 500MHz): 7.18 (4H, d, ArH), 7.05 (4H, d, ArH), 5.70 (2H, split, CH), 3.80 (2H, t, CH), 2.43 (4H, m, CH₂), 1.82 (2H, m, CH), 1.64 (6H, t, CH₃), 0.90 (12H, d, CH₃). ¹³C-NMR (CDCl₃, 500MHz): 173.4, 173.3, 170.7, 170.2, 140.9, 136.7, 129.5, 127.6, 70.5, 45.2, 44.8, 30.4, 22.6, 18.4. IR: 1752 cm⁻¹ (C=O ester), 1735 cm⁻¹ (C=O acid), and 3231 cm⁻¹ (OH acid). MS: 549 [M + Na]. T_{d} = 224 °C.

**Synthesis of 6b**. Synthesis was performed using the procedure described for **6a**. Yield: 90 % (orange foam). ¹H-NMR (CDCl₃, 500MHz): 7.70 (4H, t), 7.38 (4H, d, ArH), 7.15 (4H, d, ArH), 5.57 (2H, s, CH), 4.00 (2H, m, CH), 3.91(6H, s, OCH₃), 1.60 (6H, d, CH₃). ¹³C-NMR (CDCl₃, 500MHz): 173.4, 165.9, 157.9, 135.0, 134.0, 129.5, 129.1, 127.4, 127.3, 126.4, 126.3, 119.3, 105.7, 71.0, 55.5, 44.9, 18.3. IR: 1767 cm⁻¹ (C=O ester), 1748 cm⁻¹ (C=O acid), and 3447 cm⁻¹ (OH acid). MS: 597 [M + Na]. T_{d} = 235 °C.

**Proton and carbon nuclear magnetic resonance (¹H- and ¹³C-NMR).** Spectra were obtained using a Varian 500 MHz spectrometer. Samples were dissolved in deuterated chloroform (CDCl₃). Each spectrum was an average of 16 and 250 scans, respectively.

**Fourier transformed-infrared spectroscopy (FT-IR).** Spectra were obtained using a Thermo Nicolet/Avatar 360 FT-IR spectrometer. Samples solvent cast onto NaCl plates using DCM. Each spectrum was an average of 32 scans.

**Mass spectrometry (MS).** A Finnigan LCQ-DUO equipped with Xcalibur software and an adjustable API-ESI (Electrospray) Ion Source was used. Samples were dissolved in methanol and diluted to 10 µg/mL before injection using a glass syringe. Pressure during the experiments was 0.8x10⁻⁵ Torr and the API temperature 150 °C.

**Thermogravimetric analysis** (**TGA**). A Perkin-Elmer TGA7 analyzer with TAC7/DX controller equipped with a Dell OptiPlex Gx 110 computer running Perkin-Elmer Pyris software. Samples (∼10 mg) were heated under nitrogen at a rate of 10 °C/min from 25 to 400 °C. T_{d} was defined as the onset of decomposition and is represented by the beginning of a sharp slope on the thermogram.

### RESULTS

Current systems to deliver **1** or **2** have lowered the side effects associated with the drug and increased the duration of the drug. However, they achieve low drug loading with a burst drug release, or use a non-biodegradable polymer. The initial steps in the development of a biodegradable polymer that could be used to deliver **1** and **2** in a controlled and sustained manner are described herein. Thus the side effects associated with these drugs can be reduced, the duration of the drug effect can be increased, and the drug release can be localized.

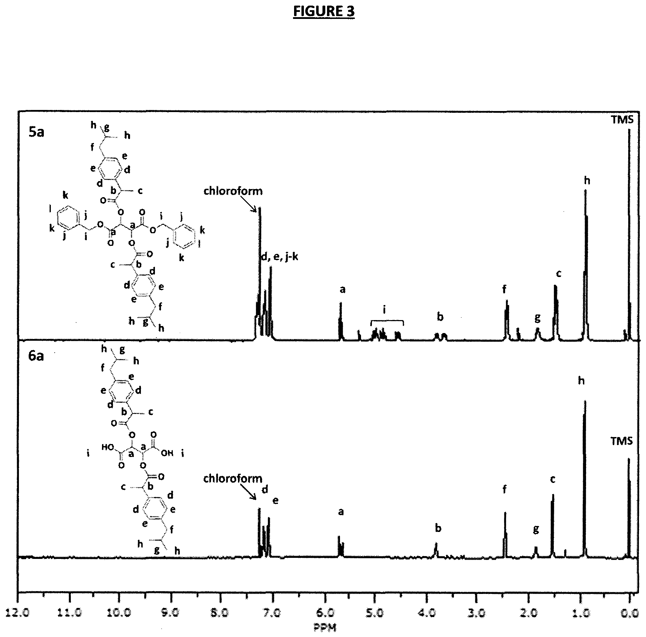

The procedure published by Lamidey et.al. (Helvetica Chimica Acta 2002, 85 (8), 2328-2334) for the synthesis of chicoric acid was adapted to synthesize compounds **5a** and **5b** and the polymer precursors **6a** and 6b, shown in Scheme 5 above. This synthetic procedure was chosen because of the structural similarities between chicoric acid and the diacids **6a** and **6b.** For the synthesis **of 5a** and **5b**, benzyl-protected tartaric acid **(4)** was coupled to the respective NSAID using EDCI (first step Scheme 5). Selective deprotection to obtain the diacids **6a** and **6b** was performed using silane-promoted palladium-mediated hydrogenation (second step Scheme 5). This debenzylation method is known to preserve the sensitive ester linkages of the diacids. The chemical structures were confirmed by ¹H- and³C-NMR and IR spectroscopies and the molecular weights by MS. Figure 3 shows the ¹H-NMR spectra of compounds **5a** and **6a**. All the expected peaks are shown in the spectra (Figure 3 labeled a-k top, a-i bottom) and no unexpected peaks were found. This data indicates the successful coupling of the drug to the tartrate backbone and that the deprotection did not break any other bonds. When the two spectra are compared, it can be seen that the debenzylation was successful as demonstrated by the disappearance of the benzylic protons (i-k, Figure 3 top). In the case of **5b** and **6b**, the debenzylation was also demonstrated by ¹H-NMR (not shown). The ¹³C-NMR (not shown) showed the presence of all carbons and no extra peaks were observed, therefore supporting that the deprotection was successful. For further characterization, the IR spectra of **5a** and **5b** show the formation of the ester bonds by the presence of the ester carbonyls (C=O) at ∼ 1768 and 1750 cm⁻¹. The IR spectra of **6a** and **6b** show that the deprotection was successful by the presence of the ester carbonyls (C=O) at ∼ 1760 and the presence of terminal carboxylic acid C=O (∼ 1740 cm⁻¹). All compounds were viscous oils or foams and did not display melting points and decomposed at temperatures between 224-294 °C.

The polymers can be prepared by treatment of a diacid precursor (*e.g*., **6a** and **6b**) with a suitable acyl chloride in a suitable solvent (*e.g*. DMF/hexanes or DCM), in the presence of a suitable base (*e.g.* an amine base such as triethylamine) as illustrated below in Scheme 6. The "R" groups illustrated in Scheme 6 below are representative examples and the present invention also comprises diacids wherein "R" is any suitable NSAID.

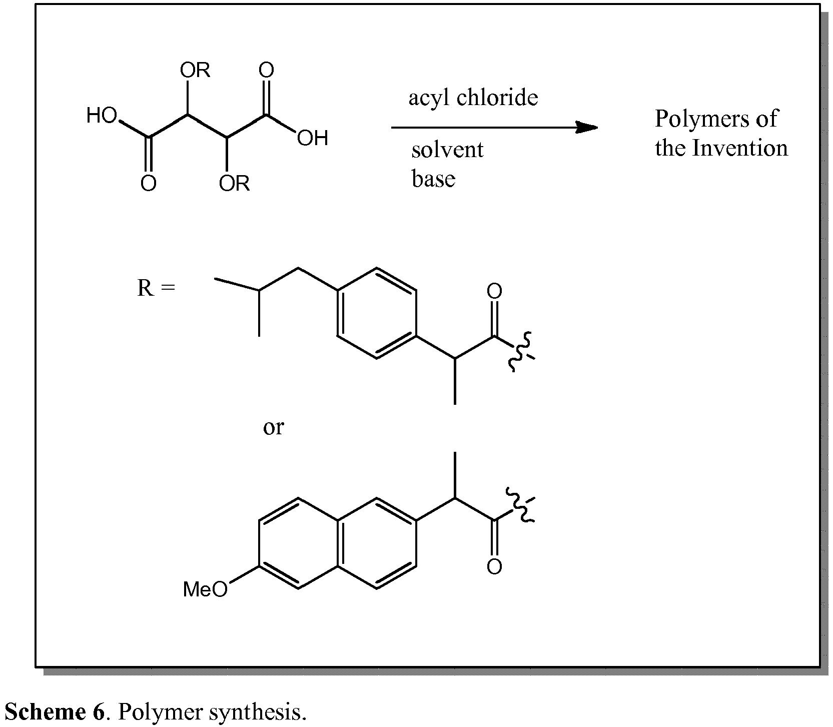

Alternatively, polycondensation may also be performed using dicyclohexylcardodiimide (DCC) coupling. A scheme showing the synthesis of a ibuprofen-based polymer using DCC coupling is shown below as an example (Scheme 7); however, polymers comprising other suitable NSAIDs may be similarly synthesized.

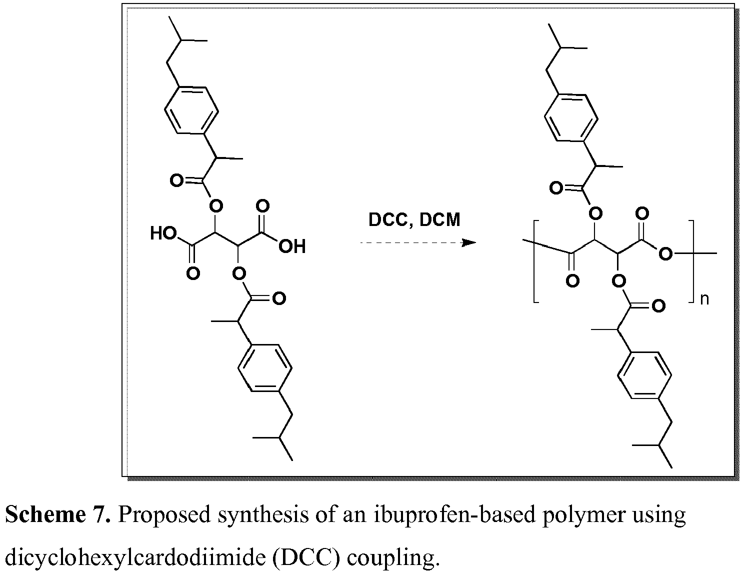

Once synthesized, physicochemical characterization of the polymers will be performed using proton and carbon nuclear magnetic resonance (¹H- and ¹³C-NMR) spectroscopies, and infrared (IR) spectroscopy. The weight-average molecular weight (M_{w}) determined by gel permeation chromatography (GPC), and the thermal properties using differential scanning calorimetry (DSC) and thermogravimetric analysis (TGA). *Furthermore, in vitro* studies will be performed to study polymer degradation and drug release in buffered media mimicking physiological conditions. Additionally, *the in vitro and in vivo* activity of the drugs released from the polymer will be studied, as well *as in vitro* cytocompatibility towards fibroblasts.

Polymers may also be formulated into microspheres using a modified procedure of a published oil-in-water single emulsion solvent evaporation technique, which is described in Examples 1 and 3.

### Example 3

Morphine is a potent narcotic analgesic used for the treatment of acute and chronic pain, and provides superior analgesia over other opioids. However, morphine has a half-life in plasma of 2-4 h, requiring repeated administration to maintain the drug at therapeutic levels for an extended time period. Repeated administration affects patient comfort because the daily activities of the patient will be interrupted in order to take the medication, which can lead to low compliance. In addition, morphine use is accompanied by the development of tolerance and dependence, leading to an increase in dosing (i.e., amount and frequency). Other side effects that can result from morphine use are respiratory depression, somnolence, and gastrointestinal effects (*e.g.*, nausea, vomiting, and constipation).

Sustained- and controlled-release morphine formulations can improve patient compliance by prolonging the analgesic effect of the drug and preventing accidental withdrawals due to missed doses. In recent years, the formulation of morphine delivery systems for sustained- and controlled-release has increased. Various delivery systems are commercially available that use enteral and parenteral administration. Among the different administration routes, enteral is the most frequently used. Among commercially available morphine delivery systems (tablets or capsules) are Kadian® (Ross, et al., International Journal of Clinical Practice, 62 (2008) 471-479; J. Chao, Pain Medicine, 6 (2005) 262-265), Avinza® (Portenoy, et al., Journal of Pain and Symptom Management, 23 (2002) 292-300; King, et al., Clinical Journal of Oncology Nursing, 7 (2003) 458), and MS Contin®(Hagen, et al., Journal of Pain and Symptom Management, 29 (2005) 80-90) that can release morphine for 12-24 h. Even though these tablets and capsules are successful at maintaining long-term benefits of the drug without dose escalation, they are also sensitive to physical alterations that destroy their release mechanism. When the tablet or capsule is crushed, chewed, or dissolved it increases the risk of administration of a fatal dose. Because they contain a large dose that can be easily separated (by crushing or breaking the tablet/capsule), they also increase the potential for recreational use. Other formulations have been extensively explored for different administration routes including lipid-based carriers (Kim, et al., Cancer Chemotherapy and Pharmacology, 33 (1993) 187-190; Grant, et al., Anesthesia & Analgesia, 79 (1994) 706-709; Wang, et al., International Journal of Pharmaceutics, 353 (2008) 95-104; Kuchler, et al., Journal of Biotechnology, 148 (2010) 24-30), drug encapsulation within a polymer (Polard, et al., International Journal of Pharmaceutics, 134 (1996) 37-46; Morales et al., Molecular Pharmaceutics, 8 (2011) 629-634; Morales, et al., Journal of Controlled Release, 95 (2004) 75-81; Arias, et al., Colloids and Surfaces B: Biointerfaces, 70 (2009) 207-212), and polymer-drug complexes (Fernández-Arévalo, et al., AAPS PharmSciTech, 5 (2004) e39; Holgado, European Journal of Pharmaceutics and Biopharmaceutics, 70 (2008) 544-549; Alvarez-Fuentes, International Journal of Pharmaceutics, 139 (1996) 237-241). Previously, morphine was chemically incorporated into a polyurethane backbone (as a pendant group), however, polyurethanes are resistant to biodegradation under physiological conditions, limiting their biological potential (Mahkam, et al., Polymer Degradation and Stability, 80 (2003) 199-202). The major drawbacks of these formulations are the low drug loading achieved and/or the rapid drug release (usually evidenced by a burst release).

The chemical incorporation of drugs into poly(anhydride-ester) (PAE) backbones could solve most of the drawbacks associated with the sustained- and controlled-release formulations mentioned above. In the last decade multiple non-steroidal anti-inflammatory drugs (*e.g.,* salicylic acid) and antiseptics (*e.g.,* catechol) have been chemically incorporated into PAE backbones (Erdmann, et al., Biomaterials, 21 (2000) 1941-1946; Schmeltzer, et al., Polymer Bulletin, 49 (2003) 441-448; Schmeltzer, et al., Biomacromolecules, 6 (2004) 359-367; Prudencioet al., Macromolecules, 38 (2005) 6895-6901; Schmeltzer, et al., Polymer Bulletin, 57 (2006) 281-291; Prudencio, et al., Macromolecular Rapid Communications, 30 (2009) 1101-1108). These new classes of polymers are capable of achieving high drug loading (50-80 %) in a reproducible manner. The drug is chemically incorporated in each repeat unit through a "linker" molecule. These PAEs release the drug in a near zero-order fashion without a burst (Whitaker-Brothers, et al., Journal of Biomedical Materials Research Part A, 76A (2006) 470-479). Drug release can be controlled and sustained by altering the chemical composition of the polymer ("linker" molecule) (Prudencio, et al., Macromolecules, 38 (2005) 6895-6901). These PAEs are also advantageous because they can be formulated into different geometries, depending on the intended administration route. For example, they can be formulated into microspheres for injectable administration (Yeagy, et al., Journal of Microencapsulation, 23 (2006) 643-653).

As described below, a morphine-based PAE was designed to control and sustain morphine release to achieve analgesia. In this work, the synthesis, characterization, and *in vitro and in vivo* analysis of this morphine-based PAE (also referred to as PolyMorphine) is presented. The polymer was synthesized by melt-condensation polymerization and the physicochemical characterization performed using proton and carbon nuclear magnetic resonance (¹H- and ¹³C-NMR) spectroscopies, and infrared (IR) spectroscopy. The weight-average molecular weight (M_{w}) was determined by gel permeation chromatography (GPC), and the thermal properties were determined using differential scanning calorimetry (DSC) and thermogravimetric analysis (TGA). *Furthermore, in vitro* studies were performed to study polymer degradation and drug release in buffered media mimicking physiological conditions, and cytocompatibility towards fibroblasts. *In vivo* studies were performed using mice to determine the analgesic effect and tolerance development using tail-flick latency (TFL) test.

### Materials and Methods

*Chemical and reagents.* Morphine was kindly provided by Noramco Inc. (Athens, GA). Acetic anhydride used to synthesize the polymer was purchased from Fischer (Fair Lawn, NJ). All other chemicals and reagents were purchased from Sigma-Aldrich (Milwaukee, WI).

*¹H-NMR and* ¹³*C-NMR and IR spectroscopies.* ¹H- and ¹³C-NMR spectra were obtained using a Varian 500 MHz spectrometer. Samples were dissolved (∼ 5 mg/mL for ¹H-NMR and ∼ 20 mg/mL for ¹³C-NMR) in deuterated dimethyl sulfoxide (DMSO-*d₆*), which was used as an internal reference. Each spectrum was an average of 16 and 250 scans, respectively.

Fourier transform infrared (FT-IR) spectra were obtained using a Thermo Nicolet/Avatar 360 FT-IR spectrometer. Samples (1 %) were ground with KBr and compressed into a disk (13 mm diameter x 0.5 mm thick) using a hydraulic press (Carver model M) applying pressure (10,000 psi) for 1 min or solvent-casted onto NaCl plates using dichloromethane (DCM). Each spectrum was an average of 32 scans.

*Molecular weight.* Mass spectrometry (MS) was used to determine the molecular weight (MW) of polymer intermediates. A Finnigan LCQ-DUO equipped with Xcalibur software and an adjustable API-ESI (Electrospray) Ion Source was used. Samples were dissolved in methanol and diluted to 10 µg/mL before injection using a glass syringe. Pressure during the experiments was 0.8x10⁻⁵ Torr and the API temperature 150 °C.

GPC was used to determine the M_{w} of polymer. A Perkin-Elmer LC system consisting of a Series 200 refractive index detector, a Series 200 LC pump, and an ISS 200 advanced sample processor was used. A Dell OptiPlex GX110 computer running Perkin-Elmer TurboChrom 4 software was utilized for data collection and control. The connection between the LC system and the computer was made using a Perkin-Elmer Nelson 900 Series Interface and 600 Series Link. Samples were dissolved in DCM (10 mg/mL) and filtered through 0.45 µm polytetrafluoroethylene syringe filters (Fischer) prior to elution through a Jordi divinylbenzene mixed-bed GPC column (7.8 x 300 mm) (Alltech Associates, Deerfield, IL) at a rate of 1 mL/min for a total run time of 30 min. M_{w} were calculated relative to narrow M_{w} polystyrene standards (Polysciences, Dorval, Canada).

*Thermal analysis.* Thermal analysis was performed using DSC to obtain the glass transition (Tg) and melting (Tₘ) temperatures. DSC was performed using a Thermal Advantage (TA) DSC Q200 running on an IBM ThinkCentre computer equipped with TA Instrument Explorer software for data collection and control. Samples (4-8 mg) were heated under nitrogen from -10 °C to 200 °C at a heating rate of 10 °C/min. A minimum of two heating/cooling cycles were used for each sample set. TA Instruments Universal Analysis 2000, version 4.5A was used to analyze the data.

TGA was used to obtain the decomposition temperatures (T_{d}). TGA analysis was performed using a Perkin-Elmer TGA7 analyzer with TAC7/DX controller equipped with a Dell OptiPlex Gx 110 computer running Perkin-Elmer Pyris software. Samples (∼10 mg) were heated under nitrogen at a rate of 10 °C/min from 25 to 400 °C. T_{d} was defined as the onset of decomposition and is represented by the beginning of a sharp slope on the thermogram.

*Diacid synthesis (3).* Morphine (**1**, 1.00 g, 1 eq) was dissolved in anhydrous pyridine under argon and stirred for 5 min. Glutaric anhydride (2, 3.97 g, 10 eq) was slowly added manually. The reaction mixture was heated to 60 °C and stirred overnight. Pyridine was azeotropically removed using toluene. The brown paste obtained was washed 10 x 50 mL with DCM to remove the excess glutaric acid. The final product was dried under vacuum at room temperature. Yield: 0.95 g (95 %) beige foam. ¹H-NMR (500 MHz, DMSO-d₆, δ): 6.73 (d, 1H, ArH), 6.58 (d, 1H, ArH), 5.50 (dq, 2H, CH and CH), 5.15 (s, 1H, CH), 5.05 (d, 1H, CH), 3.37 (s, 1H, CH₂), 2.98 (d, 1H, CH), 2.75 (s, 1H, CH), 2.40-2.15 (comp, 14H, 5CH₂ and CH₃), 2.08 (t, 1H, CH₂), 1.86-1.68 (comp, 4H, CH₂ and CH₂), 1.65 (d, 1H, CH₂). ¹³C-NMR (500 MHz, DMSO-d₆, δ): 174.1 (2C), 171.9 (1C), 170.5 (1C), 149.1 (1C), 131.5 (1C), 130.5 (1C), 130.3 (1C), 129.2 (1C), 127.8 (1C), 122.5 (1C), 119.7 (1C), 87.9 (1C), 67.4 (1C), 58.8 (1C), 45.8 (1C), 41.4 (1C), 40.8 (1C), 36.6 (1C), 32.9 (1C), 32.8 (1C), 32.6 (3C), 32.3 (1C), 32.9 (1C), 20.0 (1C). IR (KBr pellet): 1732 cm⁻¹ (C=O, ester), 1712 cm⁻¹ (C=O, acid). MS: 514 [M + 1]. T_{d} = 227 °C.

*Monomer synthesis (4).* Morphine-based diacid (3, 0.18 g) was acetylated by reacting with an excess of acetic anhydride (36 mL). The reaction mixture was stirred overnight at room temperature. The excess acetic anhydride was removed under reduced pressure. Yield: 0.16g (89 %), orange paste. ¹H-NMR (500 MHz, DMSO-d₆, δ): 6.74 (d, 1H, ArH), 6.59 (d, 1H, ArH), 5.50 (dq, 2H, CH and CH), 5.18 (s, 1H, CH), 5.05 1H, CH), 5.05 (d, 1H, CH), 3.30 (s 1H, CH₂), 2.97 (d, 1H, CH), 2.78-2.12 (comp, 20H, CH, 5CH₂ and 3CH₃), 2.05 (t, 1H, CH₂), 1.96-1.77 (comp, 4H, CH₂ and CH₂), 1.62 (d, 1H, CH₂). ¹³C-NMR (500 MHz, DMSO-d₆, δ): 172.2 (2C), 170.8 (2C), 169.2 (1C), 168.8 (1C), 145.0 (1C), 132.5 (1C), 132.3 (1C), 131.2 (1C), 131.1 (1C), 128.4 (1C), 122.3 (1C), 119.8 (1C), 89.7 (1C), 69.2 (1C), 58.4 (1C), 46.5 (1C), 43.4 (1C), 43.3 (1C), 35.4 (1C), 34.3 (1C), 34.2 (1C), 32.9 (3C), 32.5 (1C), 32.0 (1C), 30.0 (2C), 20.0 (1C). IR (solvent-casted DCM): 1809 cm⁻¹ and 1761 cm⁻¹ (C=O, anhydride), 1732 cm⁻¹ (C=O, ester). MS: 598 [M + 1]. Tₘ = 164 °C. T_{d} = 297 °C.

*Polymer synthesis (5).* Morphine-based monomer (**4**, 1.00 g) was polymerized by melt-condensation polymerization at 170 °C, under constant vacuum (< 2 mmHg), and constant stirring (100 rpm) using an overhead mechanical stirrer (T-line laboratory stirrer, Talboys Engineering Corp., Montrose, PA). Polymerization continued until the mixture solidified (∼ 30 min). The product was cooled down to room temperature and dissolved in DCM (2 mL). The polymer was isolated by precipitation over excess diethyl ether (50 mL), then isolated by vacuum filtration. The product was dried under vacuum at room temperature overnight. Yield: 0.70 g (70 %), tan solid. ¹H-NMR (500 MHz, DMSO-d₆, δ): 6.71 (br 1H, ArH), 6.55 (br, 1H, ArH), 5.50 (br, 2H, CH and CH), 5.15 (br, 1H, CH), 5.05 (br, 1H, CH), 3.29 (br, 1H, CH₂), 2.93 (br, 1H, CH), 2.76-2.17 (br, 15H, 6CH₂ and CH₃) 2.00 (br, 1H, CH₂), 1.93-1.67 (br, 4H, CH₂ and CH₂), 1.68 (br, 1H, CH₂). ¹³C-NMR (500 MHz, DMSO-d₆, δ): 175.1 (1C), 172.8 (1C), 172.6 (1C), 171.2 (1C), 149.9 (1C), 133.5 (1C), 132.4 (1C), 131.9 (1C), 130.8 (1C), 129.0 (1C), 122.5 (1C), 120.0 (1C), 89.3 (1C), 68.8 (1C), 58.8 (1C), 46.8 (1C), 43.5 (1C), 43.1 (1C), 35.4 (1C), 33.8 (1C), 33.5 (1C), 33.1 (3C), 32.9 (1C), 20.9 (1C). IR (solvent-casted DCM): 1818 cm⁻¹ and 1761 cm⁻¹ (C=O, anhydride), 1734 cm⁻¹ (C=O, ester). M_{w}= 26,100 Da, PDI = 1.14. Tg = 120 °C. T_{d} = 185 °C.

*High-performance liquid chromatography (HPLC).* Quantitative analysis of *the in vitro* degradation products was performed *via* HPLC using an XTerra® RP18 5 µm 4.6 x 150 mm column (Waters, Milford, MA) on a Waters 2695 Separations Module equipped with a Waters 2487 Dual λ Absorbance Detector. The system was connected to a Dell computer running Empire software. Samples were filtered using 0.22 µm poly(vinylidine fluoride) syringe filters (Fisher). HPLC method was adapted from previously published methods (Gerostamoulos, et al., Forensic Science International, 77 (1996) 53-63; Meng, et al., Journal of Chromatography B: Biomedical Sciences and Applications, 742 (2000) 115-123). Mobile phase used was composed of 50 mM KH₂PO₄, 2.5 mM sodium dodecyl sulfate, 25 % acetonitrile, 75% water at pH 3. Samples (20 µL) were run at 35 °C at a flow rate of 1 mL/min. Morphine was monitored at λ = 210 nm. The instrument was calibrated using standard morphine 1 and diacid 3 solutions of known concentrations.

In vitro *degradation studies.* Diacid **3** (5.0 mg, triplicate) was placed into scintillation vials and 20.00 mL phosphate buffered saline (PBS) pH 7.4 added. Samples were incubated at 37 °C under constant shaking (60 rpm) in an Excella E25 Incubator Shaker (New Brunswick Scientific). PBS (1.00 mL) was removed at predetermined time points (2 h, 5 h, 10 h, and daily starting on day 1) and replaced with fresh PBS (1.00 mL). The pH was checked using an Accumet® Research AR15 pH meter (Fisher Scientific) and adjusted using 0.50 M NaOH when needed. Samples were immediately analyzed by HPLC using the conditions outlined above.

For the polymer degradation studies, polymer **5** (5.0 mg, triplicate) was placed into scintillation vials and 20.00 mL phosphate buffered saline (PBS) pH 7.4 added. Samples were incubated at 37 °C under constant shaking (60 rpm) in an Excella E25 Incubator Shaker (New Brunswick Scientific). PBS (20.00 mL) was removed daily and replaced with fresh PBS. Samples were analyzed by HPLC.

*Microsphere Formulation.* Polymer **5** was formulated into microspheres using a modified procedure of a published oil-in-water single emulsion solvent evaporation technique. In general, polymer **5** (0.098 g) were dissolved in dichloromethane (1 mL) and added drop-wise to 1% aqueous poly(vinyl alcohol) (PVA) solution (30 mL) at room temperature. The emulsion was homogenized for 2 min using an IKA Ultra-Turrax T8 homogenizer at approximately 10,000 rpm. The homogenized solution was left stirring for 2 h to allow microsphere formation by solvent evaporation. Microspheres were transferred to sterile 50 mL polypropylene conical tubes (30 x 115 mm style, BD Falcon, Franklin Lakes, NJ), washed with acidic water (pH 1) to remove residual PVA, and isolated by centrifugation at 3,000 rpm for 10 min. Microspheres were frozen by placing the conical tubes in a dry ice/acetone bath and lyophilized for 24 h at -40 °C and 133x10⁻³ mBar (LABCONO Freeze Dry System/Freezon 4.5).

*Cell cytocompatibility studies.* Cytocompatibility was evaluated by culturing 3T3 fibroblasts cells (NIH 3T3 fibroblast cell line) in diacid- and/or polymer-containing medium at concentrations of 0.10 and 0.01 mg/mL Cell culture medium consisted of Dulbecco's modified Eagle's medium (DMEM), 10 vol.% fetal bovine serum (Atlanta Biologicals, Lawrenceville, GA), 1% 1-glutamate, and 1% penicillin/streptomycin. Fibroblasts were seeded at a density of 2,000 cells/well in 96 well plates containing 150 µL of culture medium. The positive control consisted of fibroblasts with cell culture media only and the negative control consisted of fibroblasts with cell culture media and 5% 200-proof ethanol (PHARMCO-AAPER). Cells were incubated at 37 °C and 5% CO₂ for 24, 48 and 72 h. Cell viability was determined by using Calcein AM and ethidium homodimer-1 staining (Molecular Probes) according to the manufacturer's protocol and the results normalized to the positive control. For each of the three time points (24, 48 and 72 h), a student's t-test was performed to assess for statistical significance between the positive control and experimental conditions. Experiments were performed in quadruplicates.

In vivo *animal studies.* Adult male C57B1/6J mice were obtained from Charles River (Kingston, NY). Animals were approximately 10 weeks old and weighed between 19.5 - 27.7 g at the beginning of the study. Animals were housed in climate-controlled rooms with a 12:12 hour light/dark cycle, with food and water available *ad libitum.* All animal procedures were approved by the Institutional Animal Care and Use Committee (IACUC) at Rutgers University, and consistent with the Guide for the Care and Use of Laboratory Animals (National Institutes of Health, 2011).

Animals were pre-handled twice a day for 3 days prior to the experiment. Polymer 5 (200.0 mg powder) was suspended in 10 mL of 5 % Cremophor EL in saline by vortex and stirred for 15 min. Diacid 3 (50.0 mg foam) and morphine HCl (10 mg) were each dissolved in 10 mL of 5 % Cremophor EL in saline. A 5 % Cremophor EL saline solution was used as the vehicle control. All administrations were by intraperitoneal (i.p.) injection. Drug dosing was as following: free morphine (morphine HCl) at 10 mg/kg, **3** at 50 mg/kg, and **5** at 200 mg/kg.

Nociception in mice was measured with the TFL test. Animals were wrapped loosely in soft cloth, where each cage of animals had its own cloth to minimize cross-cage olfactory sensory stimulation. TFL was tested by immersing the distal third of the animal's tail in a water bath at 49 °C, and the TFL time was recorded with a 30-sec cutoff time to avoid tissue damage. Animals were only tested one time at each time point.

There were 30 animals in each drug group at the beginning of the study. TFL was measured at the following time points after the drug administration: 30 min, 1 h, 2 h, 4 h, 8 h, 1 d, 2 d, 3 d, 7 d, 9 d, and 14 d. On day 3, 15 animals from each group (including the vehicle control group) were tested for morphine sensitivity by being subjected to an acute morphine dose (10 mg/kg of free morphine). The remaining 15 animals continued to be tested as scheduled. On day 14, after being tested for TFL, all animals received an acute dose of morphine (10 mg/kg of free morphine) and tested for morphine sensitivity.

### Results

*Synthesis and physicochemical characterization.* In an effort to overcome the limitations of commercially available morphine delivery systems, a morphine-based poly(anhydride-ester) (PAE), described herein as PolyMorphine (**5**), was developed and evaluated. The synthesis of this polymeric prodrug consists of three steps as outlined in **Scheme 8** below: esterification of morphine to yield the diacid (**3**), which is then activated via acetylation to form the monomer (**4**) that undergoes melt-condensation polymerization to yield polymer (**5**). All compounds synthesized were characterized to assess their physical and chemical properties. Their chemical structures were assessed using ¹H- and ¹³C-NMR, and FT-IR spectroscopy. ¹³C-NMR was also used to confirm the preservation of morphine's structural integrity throughout the synthetic procedures. The MW and M_{w} were determined with MS and GPC, respectively. The thermal properties were evaluated using DSC and TGA.

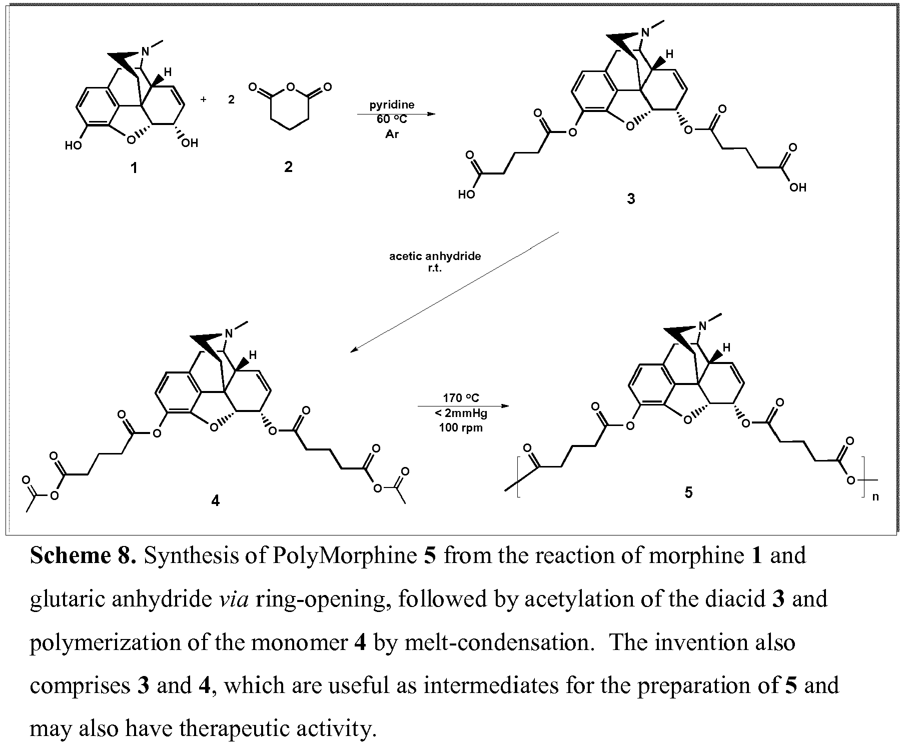

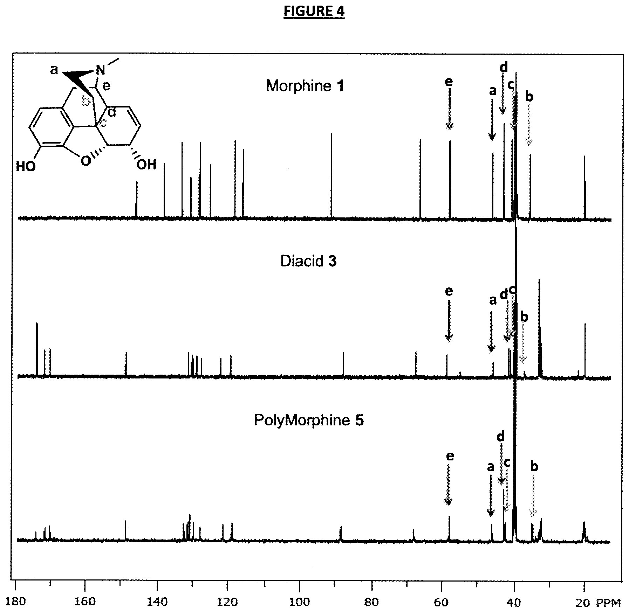

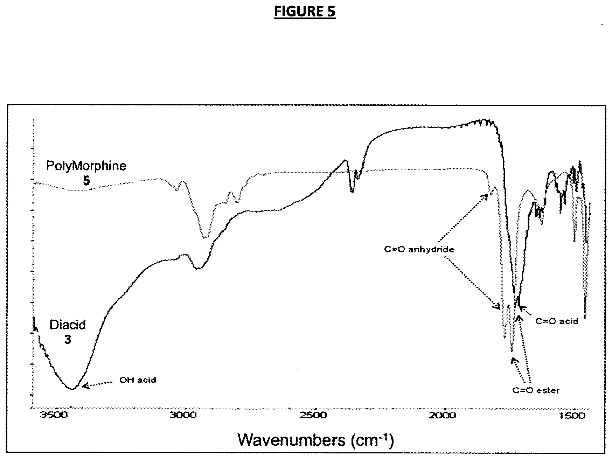

To synthesize **3**, various reaction conditions were explored by changing the solvent and the base catalyst. Among the conditions tested, the reaction carried out neat in pyridine yielded the best results (i.e., full conversion into product and easy product isolation). Because the phenolic hydroxyl group of morphine is more reactive than the allylic alcohol, the conversion of both alcohols takes 3 days for completion at room temperature. When heated to 60 °C, esterification of the phenollic and allyilc alcohols complete within 24 hours. The isolation of the product was performed by azeotropic removal of pyridine with toluene to reproducibly produce **3** in high yields (95 %). Figure 4 shows the ¹³C-NMR of **1**, **3**, and **5**; the key peaks for the nitrogen-containing ring are indicated. As shown in Figure 4, the structure of the nitrogen-containing ring of the drug was preserved in **3.** The IR spectrum of **3** (Figure 5) shows the attachment of glutaric likers by the formation of the ester bonds by the presence of the ester carbonyl (C=O) at 1732 cm⁻¹ and the presence of terminal carboxylic acid C=O at 1712 cm⁻¹. The MW of 3 was determined to be 514 [M + H⁺], by MS. The thermal analysis of **3** shows that it decomposes at 227 °C and did not display a Tₘ.

Two different polymerization methods were investigated to prepare PolyMorphine. Given a concern that morphine intermediates would be thermally unstable, solution polymerization was first evaluated. This method uses triphosgene (which forms *phosgene in situ*) as the coupling agent in the presence of triethylamine (Schmeltzer, et al., Journal of Biomaterials Science, Polymer Edition, 19 (2008) 1295-1306). However, this polymerization method not only resulted in low M_{w} polymer and low yields, but the pure polymer could not be isolated either. In contrast, melt-condensation polymerization is known to result in higher yields, higher M_{w} product, and pure polymer (Schmeltzer, et al., Journal of Biomaterials Science, Polymer Edition, 19 (2008) 1295-1306). In addition, melt-condensation is reproducible and amenable to scale-up, from milligrams to tens of grams. Monomer **4** was prepared by the acetylation of **3** in excess acetic anhydride at room temperature. Characterization of **4** was performed with the same methods used to characterize **3**. Monomer **4** decomposes at 297 °C and melts at 164 °C, this high T_{d} of **4** and its moderate Tₘ made possible the polymerization by melt-condensation polymerization because it was thermally stable.

Melt-condensation polymerization of activated **4** at 170 °C *in vacuo* yielded **5** with reasonably high M_{w} (26,000 Da), low PDI (1.1) and high yields (70 %). The IR spectrum of **5** (Figure 5) show the formation of the anhydride bonds by the presence of the anhydride C=O at 1818 and 1761 cm⁻¹, the preservation of the ester bonds by the presence of the ester C=O at 1734 cm⁻¹, and the disappearance of terminal carboxylic acid C=O at 1712 cm⁻¹. Figure 4 also shows the ¹³C-NMR spectrum of **5**, as seen on the figure the structure of the drug was preserved. PolyMorphine **5** decomposes at 185 °C, does not have a Tₘ, and its Tg is 120 °C. Having such a high T_{g} is a positive attribute *for in vivo* applications (i.e., body temperature is 37 °C) because the polymer will not deform once implanted in the body.

*In vitro degradation and drug release.* Given that **5** was designed to degrade and release free *morphine, in vitro* hydrolysis studies were performed to monitor polymer degradation. Since the hydrolytic cleavage of the anhydride bonds is faster than the ester bonds (Achim, Biomaterials, 17 (1996) 103-114; Siepmann, et al., Advanced Drug Delivery Reviews, 48 (2001) 229-247), the degradation of the **3** was expected to be the rate-determining step in the degradation of **5**. In addition, the two ester bonds in compound **3** are not equivalent and would likely degrade at different rates. Diacid **3** is an important intermediate; if it does not degrade to release free morphine, then polymer **5** may not degrade into free morphine.

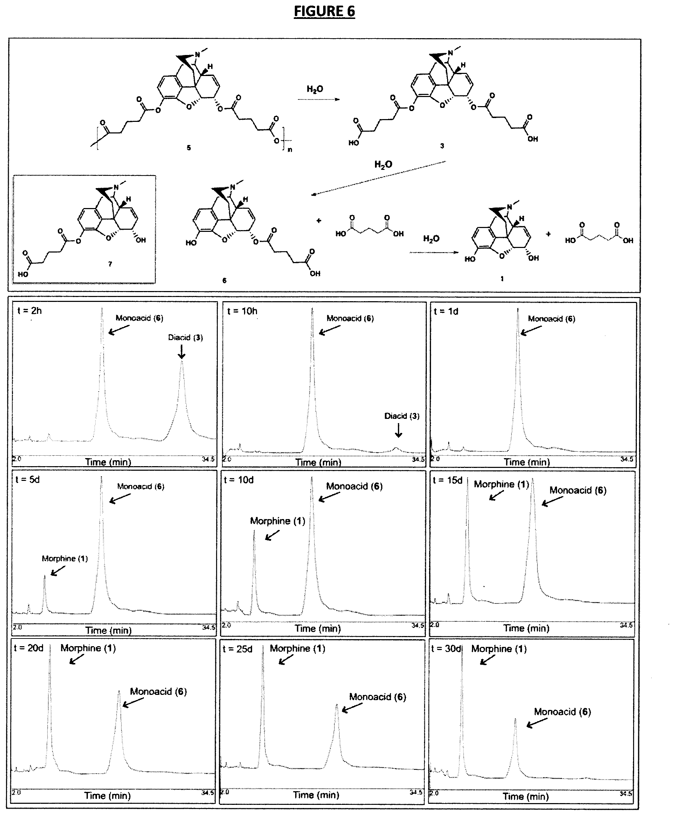

Mimicking physiological conditions (37 °C and pH 7.4 buffer), the hydrolytic degradation **of 3** was analyzed by HPLC where three distinctive peaks were detected: **3** (Rₜ = 28.5 min), **5** (Rₜ = 16.2 min), and **1** (Rₜ = 6.5 min). Diacid **3** hydrolyzes into a monoacid (Figure 6 top, **6**), which then hydrolyzes into free morphine. The formation of **6** during degradation was confirmed by the analysis of monoacid **7** (that was synthesized by performing the diacid synthesis for 1 day at room temperature). The retention time of **7** was 18.1 min, which is different from that of **6**. When both monoacids were analyzed simultaneously, a peak with two maximums was observed; the low resolution suggests the presence of two similar compounds. Figure 6 (bottom) shows representative chromatograms for the degradation of **3** into the intermediate **6** and **1**.

Following analysis of the **3**, the hydrolytic degradation of **5** was studied under similar conditions. The HPLC results indicated that the polymer degrades via hydrolytic cleavage of the anhydride bonds to generate **3**, which is then hydrolyzed into **6**, which further hydrolyzes into **1** (Figure 6 top).

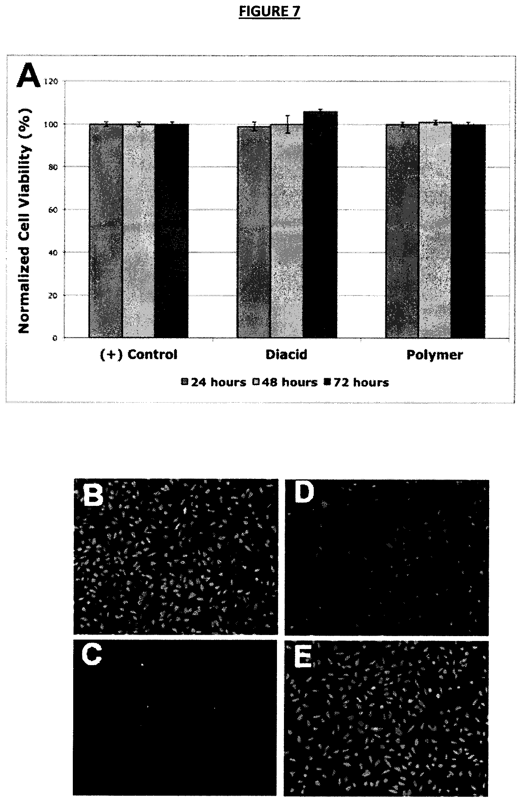

In vitro *cytocompatibility.* Investigating the potential toxicity of these novel materials is critical to understanding the *potential in vivo* use of this prodrug. The cytotoxicity of **3** and **5** towards fibroblasts was *studied in vitro*. Fibroblasts were used for this study because they are the most frequently used cells for initial cytotoxicity testing of biomaterials. Cytocompatibility was evaluated by culturing 3T3 fibroblasts cells in medium containing **3** and **5** (separately) at concentrations of 0.10 and 0.01 mg/mL. These concentrations were chosen because they are well above the concentrations *seen in vitro* (10-100 times higher) and can be used to determine a possible dose dependent toxicity. Studies were performed evaluating cell viability at 24, 48, and 72 h, to evaluate early and late degradation stages. To quantify cell viability, representative fluorescence microscopy images of each condition were taken to determine the total number of cells (live and dead). Statistical analysis showed no significant differences with a 95 % confidence level between the samples containing **3** and **5** and the positive control for both concentrations used at all time points. Comparison between the diacid- and polymer-containing samples and the media control indicate normal to higher cell viability suggesting that both **3** and **5** are non-cytotoxic (Figure 7A). Figure 7 (B-D) shows representative fluorescence microscopy images of the positive control (fibroblasts with cell culture media), the negative control (fibroblasts with cell culture media and 5 % ethanol), cell culture media containing **3** (0.10 mg/mL at 48 hours), and cell culture media containing **5** (0.10 mg/mL at 48 hours). These results show no significant cytotoxicity caused by **5** or **3** (Figure 7D & E), whereas fluorescence from the negative control showed dead cells (Figure 7C).

In vivo *evaluation: analgesic effect and tolerance development.* As outlined above, a key motivator of this work was to develop a prodrug form of morphine (PolyMorphine), which when *administered in vivo,* would hydrolytically degrade in a controlled fashion to provide extended analgesia. To determine whether 5 would meet this objective, mice were systemically administered morphine (i.p. injection) and their nociception measured using the TFL test. TFL was tested by immersing the distal third of the animal's tail in a water bath at 49 °C. Four treatment groups were used: vehicle control, free morphine (at 10 mg/kg), **3** (at 50 mg/kg), and **5** (at 200 mg/kg). At various time points post administration (starting after 30 min), TFL was measured.

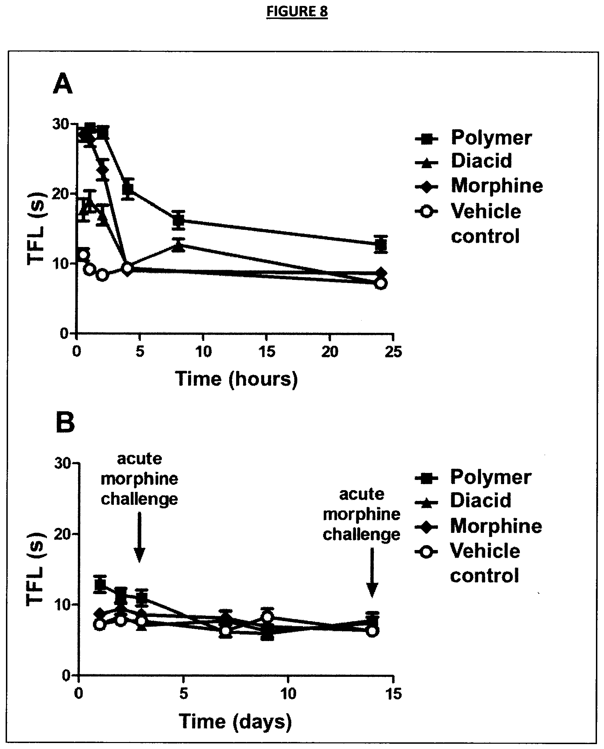

As shown in Figure 8, free morphine provided strong analgesia, peaking 30 min post-administration (Figure 8A, filled diamonds). The analgesic effect of free morphine diminished with time; by the 4 h time point, the analgesic effect was completely absent. This time course of analgesia has been well-established for free morphine, as the drug is *metabolized in vivo* and plasma drug level drops off (Olsson, et al., International Journal of Pharmaceutics, 119 (1995) 223-229). The **3** showed a similar analgesic effect as free morphine; analgesia diminished by 8 h.

I.P. injection **of 5** (as a suspension) also provided strong analgesia, reaching a peak effect at the 1 h time point (Figure 8A, filled squares). Different from free morphine, however, is the noticeably extended time course of analgesic effect from PolyMorphine. Analgesia was sustained throughout the 24 h time frame post drug administration with gradual decline (Figure 8A), with the analgesic effect still detectable 3 days post-administration (Figure 8B). These results clearly indicate that **5**, when *administered in vivo,* provides extended pain relief. The fact that analgesia was detectable 3 days post-administration was remarkable; this study is the first example of a single dose, systemically administered morphine formulation that displayed analgesia for over 24 h.

In opioid biology, a well-known effect of the extended use of morphine (and related opioid alkaloids with strong analgesic properties) is tolerance development with repeated exposure. Thus, when morphine is given either by repeated administrations to a subject (rodent or human) over days, or by surgical embedding of a morphine pellet in a laboratory animal, morphine tolerance invariably develops, manifested as reduced effectiveness of morphine-induced analgesia. Given that we are developing an extended release version of morphine, we needed to test for morphine tolerance development in animals that received **5**, as its analgesic effect lasted several days. If the animals become tolerant to morphine, it is expected that they would be non-responsive or would flick their tails in less than 30 s (cutoff time) when their tails are immersed in the hot water. Two time points were chosen at which the animal's responsiveness to an acute morphine challenge was tested. The first time point was 3 days post-administration, as this was the time when PolyMorphine's analgesic effect has decreased to near baseline level. Half of the mice from each drug group were subjected to acute morphine challenge on day 3. The remaining half of the mice from each experimental group were subjected to acute morphine challenge on day 14.

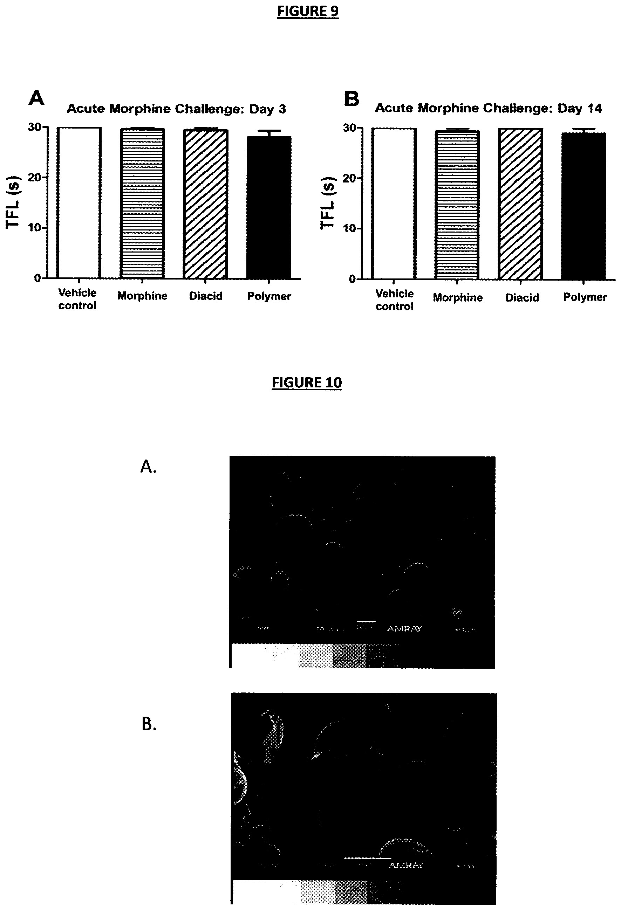

As shown in Figure 9, mice in every group showed full responsiveness to acute morphine challenge, at either day 3 (Figure 9A) or day 14 (Figure 9B), reaching the 30 s cutoff time 30 min morphine post-administration. No significant statistical difference was noted between the **5** group and the comparison groups at either time point (p > 0.05 for PolyMorphine vs. vehicle control, free morphine, or diacid group). These results suggest that even though **5** provided sustained analgesia over several days, no apparent signs of morphine tolerance development were observed.

*Microsphere Formulation.* Polymer **5** was also formulated into microspheres using a modified procedure of a published oil-in-water single emulsion solvent evaporation technique. Microspheres ∼ 10µm in diameter and with a non-smooth surface were obtained, as shown by scanning electron microscopy images in Figure 10.

In summary, this study reports the preparation and evaluation of PolyMorphine, a polymer version of morphine that provides extended analgesia while potentially reducing tolerance development. PolyMorphine was synthesized via melt-condensation polymerization and its physicochemical properties fully characterized to confirm the preservation of morphine's structural integrity. *In vitro* studies were performed to determine the degradation pathway of the polymer and a key intermediate, showing that PolyMorphine hydrolyzes into free morphine. *In vitro* cytocompatibility studies showed that PolyMoprhine is non-cytotoxic. When administered *in vivo,* PolyMorphine provided sustained pain relief for up to 3 days, more than 20 times the analgesic time window of free morphine. These results demonstrated, for the first time, a systemically administered prodrug that yields such a long-lasting analgesic effect. Furthermore, based on a preliminary test of sensitivity to an acute morphine challenge, no signs of morphine tolerance development were observed in PolyMorphine-administered animals. Furthermore, the covalent linkage of morphine molecules into PolyMorphine makes it difficult to extract morphine molecules by physical means. Thus, given the abuse liability of many controlled release formulations of opioid analgesics, PolyMorphine may offer the desirable option of a long-acting, low abuse liability alternative to conventional opioid analgesics.

### Example 4

The present invention comprises block copolymers that comprise pendant NSAIDs (e.g., ibuprofen) and morphine in the backbone. While the block copolymer may comprise any suitable pendant NSAID, the synthetic scheme that is included below shows ibuprofen as an example (Scheme 9). Block copolymers containing pendant NSAIDs (e.g., ibuprofen) and morphine in the backbone may be synthesized using melt-condensation or solution polymerization. These two polymerization methods are well known and have been applied for the synthesis of many different polyanhydrides, including homopolymers and copolymers. 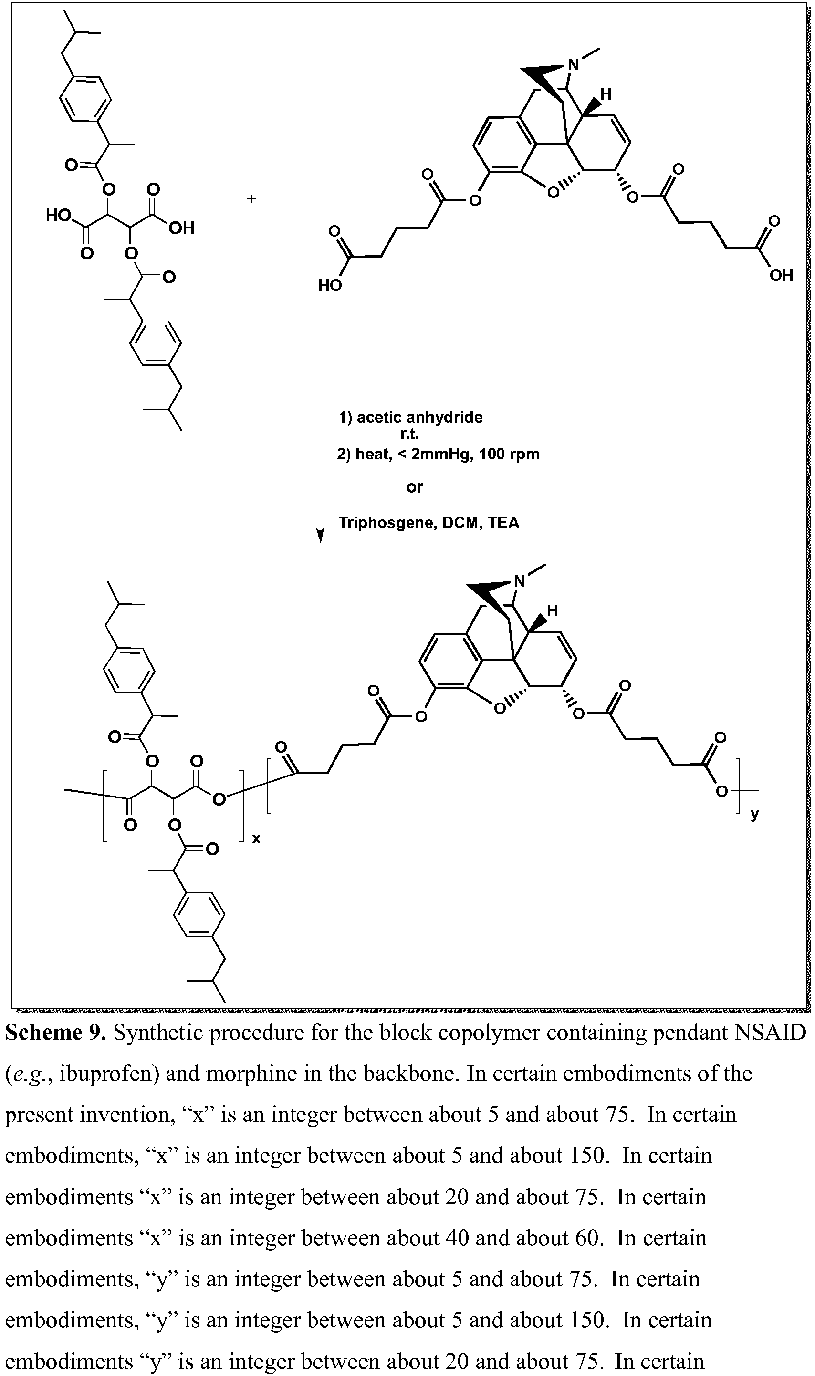 embodiments "y" is an integer between about 40 and about 60.

Once synthesized, physicochemical characterization of the polymers will be performed using proton and carbon nuclear magnetic resonance (¹H- and ¹³C-NMR) spectroscopies, and infrared (IR) spectroscopy. The weight-average molecular weight (M_{w}) determined by gel permeation chromatography (GPC), and the thermal properties using differential scanning calorimetry (DSC) and thermogravimetric analysis (TGA). *Additionally, in vitro* studies will be performed to study polymer degradation and drug release in buffered media mimicking physiological conditions and *in vitro* cytocompatibility towards fibroblasts.

Polymers may also be formulated into microspheres using a modified procedure of a published oil-in-water single emulsion solvent evaporation technique, which is described above in Examples 1 and 3.

The use of the terms "a" and "an" and "the" and similar terms in the context of describing embodiments of invention are to be construed to cover both the singular and the plural, unless otherwise indicated herein or clearly contradicted by context. The terms "comprising," "having," "including," and "containing" are to be construed as open-ended terms (*i.e.,* meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. In addition to the order detailed herein, the methods described herein can be performed in any suitable order unless otherwise indicated herein or otherwise clearly contradicted by context. The use of any and all examples, or exemplary language (e.g., "such as") provided herein, is intended merely to better illuminate embodiments of invention and does not pose a limitation on the scope of the invention unless otherwise specifically recited in the claims. No language in the specification should be construed as indicating that any non-claimed element as essential to the practice of the invention.

## Claims

1. An anhydride polymer comprising a biodegradable backbone that comprises one or more groups that will provide morphine upon hydrolysis of the polymer and one or more pendant residues of a non-steroidal anti-inflammatory.

2. The anhydride polymer of claim 1 which comprises repeating units that form the biodegradable backbone, wherein each repeating unit comprises one or more pendant residues of the non-steroidal anti-inflammatory.

3. The anhydride polymer of any one of claims 1 or 2 which comprises one or more groups of formula (I):
-C(=O)-A-C(=O)-O- (I)
wherein A is a C₁-C₈ methylene chain that is covalently linked to one or more residues of a non-steroidal anti-inflammatory through an amine, ester, amide, sulfide or ether linkage.

4. The anhydride polymer of claim 1 or 2 which comprises one or more groups of formula (II): 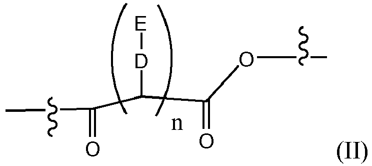 wherein each D is a direct bond, or an amine, ester, amide, sulfide, or ether linkage; each E is independently a residue that will release a non-steroidal anti-inflammatory agent upon hydrolysis of the polymer; and n is 1, 2, 3, 4, 5, 6, 7, 8, or 9.

5. The anhydride polymer of claim 4 which comprises two or more repeating groups of formula (II).

6. The anhydride polymer of claim 4 which comprises one or more groups of formula (IIa): 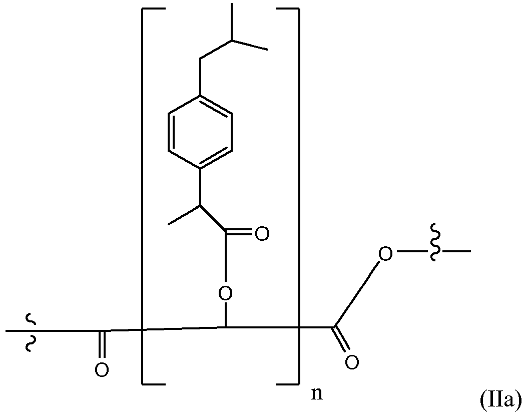 wherein n is 1, 2, 3, 4, 5, 6, 7, 8, or 9.

7. The anhydride polymer of claim 4 which comprises one or more groups of formula (IIb): 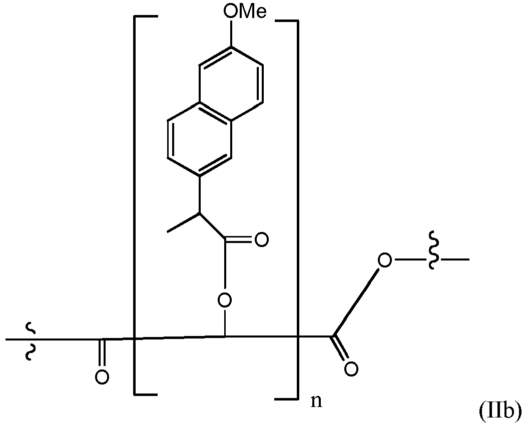 wherein n is 1, 2, 3, 4, 5, 6, 7, 8, or 9.

8. The anhydride polymer of claim 6 or 7 which comprises 2-200 repeating groups of formula (IIa) or (IIb).

9. The anhydride polymer of claim 6 or 7 which comprises at least 2, 3, 4, 5, 6, 7, 8, or 9 repeating groups of formula (IIa) or (IIb).

10. The anhydride polymer of any one of claims 1-4 wherein each non-steroidal antiinflammatory is selected from ibuprofen, naproxen, fenoprofen, ketoprofen, flurbiprofen, suprofen, benoxaprofen, indoprofen, pirprofen, carprofen, loxoprofen, pranoprofen, alminoprofen, salicylic acid, diflunisal, salsalate, oxaprozin, indomethacin, sulindac, etodolac, ketorolac, diclofenac, piroxicam, meloxicam, tenoxican, lornoxicam, isoxicam, mefenamic acid, meclofenamic acid, flufenamic acid, tolfenamic acid, lumiracoxib and licofelone.

11. The anhydride polymer of any one of claims 1-10 wherein the backbone comprises one or more groups of formula (V):

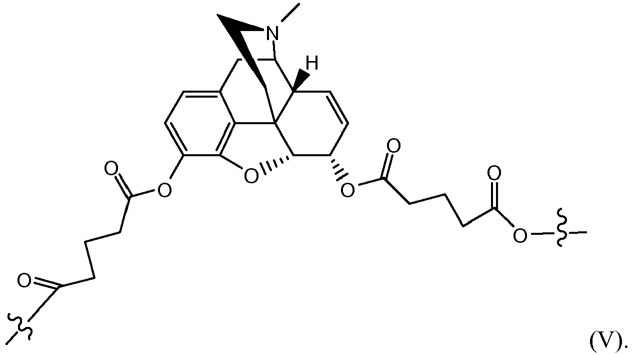

12. A microsphere that comprises an anhydride polymer of any one of claims 1-11.

13. A pharmaceutical composition comprising an anhydride polymer as described in any one of claims 1-12 and a pharmaceutically acceptable carrier.

14. An anhydride polymer as described by any one of claims 1-12 for use in medical treatment.

15. An anhydride polymer according to any one of claims 1-12 for use in treating pain.

## Patentansprüche

1. Anhydridpolymer umfassend eine biologisch abbaubare Hauptkette, die eine oder mehrere Gruppen umfasst, die bei Hydrolyse des Polymers Morphium bereitstellen, und einen oder mehrere seitenständige Reste eines nichtsteroidalen Entzündungshemmers.

2. Anhydridpolymer nach Anspruch 1, welches Wiederholungseinheiten umfasst, die die biologisch abbaubare Hauptkette bilden, wobei jede Wiederholungseinheit einen oder mehrere seitenständige Reste des nichtsteroidalen Entzündungshemmers umfasst.

3. Anhydridpolymer nach irgendeinem der Ansprüche 1 oder 2, welches eine oder mehrere Gruppen der Formel (I) umfasst:
-C(=O)-A-C(=O)-O- (I)
wobei A eine C₁-C₈-Methylenkette ist, die durch eine Amin-, Ester-, Amid-, Sulfid- oder Etherverknüpfung kovalent mit einem oder mehreren Resten eines nichtsteroidalen Entzündungshemmers verknüpft ist.

4. Anhydridpolymer nach Anspruch 1 oder 2, welches eine oder mehrere Gruppen der Formel (II) umfasst: 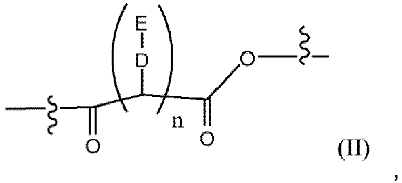 wobei jedes D eine direkte Bindung oder eine Amin-, Ester-, Amid-, Sulfid- oder Etherverknüpfung ist; jedes E unabhängig ein Rest ist, der einen nichtsteroidalen Entzündungshemmer bei Hydrolyse des Polymers freisetzt; und n 1, 2, 3, 4, 5, 6, 7, 8 oder 9 ist.

5. Anhydridpolymer nach Anspruch 4, welches zwei oder mehr Wiederholungsgruppen der Formel (II) umfasst.

6. Anhydridpolymer nach Anspruch 4, welches eine oder mehrere Gruppen der Formel (IIa) umfasst: 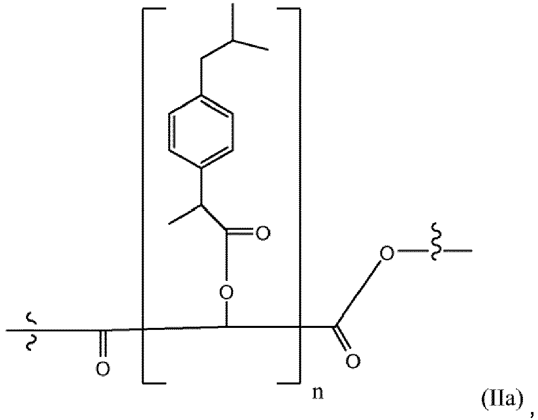 wobei n 1, 2, 3, 4, 5, 6, 7, 8 oder 9 ist.

7. Anhydridpolymer nach Anspruch 4, welches eine oder mehrere Gruppen der Formel (IIb) umfasst: 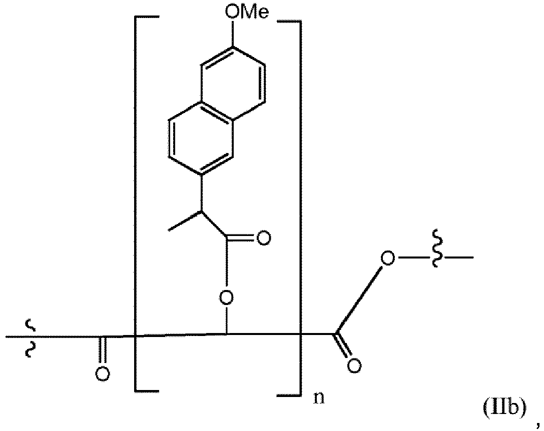 wobei n 1, 2, 3, 4, 5, 6, 7, 8 oder 9 ist.

8. Anhydridpolymer nach Anspruch 6 oder 7, welches 2-200 Wiederholungsgruppen der Formel (IIa) oder (IIb) umfasst.

9. Anhydridpolymer nach Anspruch 6 oder 7, welches mindestens 2, 3, 4, 5, 6, 7, 8 oder 9 Wiederholungsgruppen der Formel (IIa) oder (IIb) umfasst.

10. Anhydridpolymer nach irgendeinem der Ansprüche 1-4, wobei jeder nichtsteroidale Entzündungshemmer aus Ibuprofen, Naproxen, Fenoprofen, Ketoprofen, Flurbiprofen, Suprofen, Benoxaprofen, Indoprofen, Pirprofen, Carprofen, Loxoprofen, Pranoprofen, Alminoprofen, Salicylsäure, Diflunisal, Salsalat, Oxaprozin, Indomethacin, Sulindac, Etodolac, Ketorolac, Diclofenac, Piroxicam, Meloxicam, Tenoxican, Lornoxicam, Isoxicam, Mefenaminsäure, Meclofenaminsäure, Flufenaminsäure, Tolfenaminsäure, Lumiracoxib und Licofelon ausgewählt ist.

11. Anhydridpolymer nach irgendeinem der Ansprüche 1-10, wobei die Hauptkette eine oder mehrere Gruppen der Formel (V) umfasst:

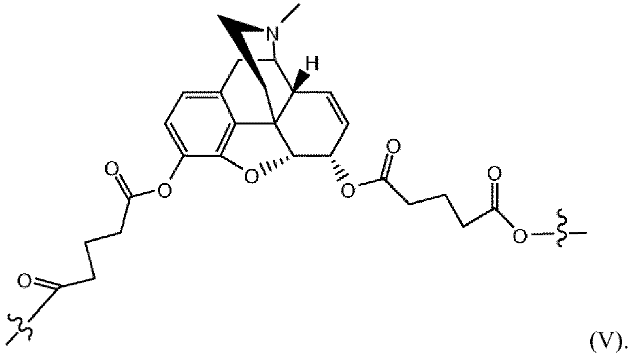

12. Mikrokugel, die ein Anhydridpolymer nach irgendeinem der Ansprüche 1-11 umfasst.

13. Pharmazeutische Zusammensetzung umfassend ein Anhydridpolymer nach irgendeinem der Ansprüche 1-12 und einen pharmazeutisch annehmbaren Träger.

14. Anhydridpolymer nach irgendeinem der Ansprüche 1-12 zur Verwendung bei einer medizinischen Behandlung.

15. Anhydridpolymer nach irgendeinem der Ansprüche 1-12 zur Verwendung bei einer Schmerzbehandlung.

## Revendications

1. Polymère d'anhydride comprenant un squelette biodégradable qui comprend un ou plusieurs groupes qui fourniront de la morphine après l'hydrolyse du polymère et un ou plusieurs résidus pendants d'un anti-inflammatoire non stéroïdien.

2. Polymère d'anhydride selon la revendication 1 qui comprend des motifs répétés qui forment le squelette biodégradable, dans lequel chaque motif répété comprend un ou plusieurs résidus pendants de l'anti-inflammatoire non stéroïdien.

3. Polymère d'anhydride selon l'une quelconque des revendications 1 ou 2 qui comprend un ou plusieurs groupes de formule (I) :
-C(=O)-A-C(=O)-O- (I)
dans laquelle A est une chaîne méthylène en C₁ à C₈ qui est liée de manière covalente à un ou plusieurs résidus d'un anti-inflammatoire non stéroïdien par une liaison amine, ester, amide, sulfure ou éther.

4. Polymère d'anhydride selon la revendication 1 ou 2 qui comprend un ou plusieurs groupes de formule (II) : 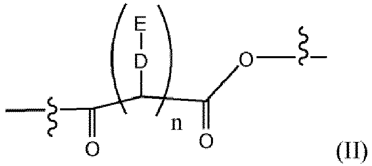 dans laquelle chaque D est une liaison directe, ou une liaison amine, ester, amide, sulfure ou éther ; chaque E est indépendamment un résidu qui libérera un agent anti-inflammatoire non-stéroïdien après l'hydrolyse du polymère ; et n vaut1, 2, 3, 4, 5, 6, 7, 8 ou 9.

5. Polymère d'anhydride selon la revendication 4 qui comprend deux ou plus de deux groupes répétés de formule (II).

6. Polymère d'anhydride selon la revendication 4 qui comprend un ou plusieurs groupes de formule (IIa) : 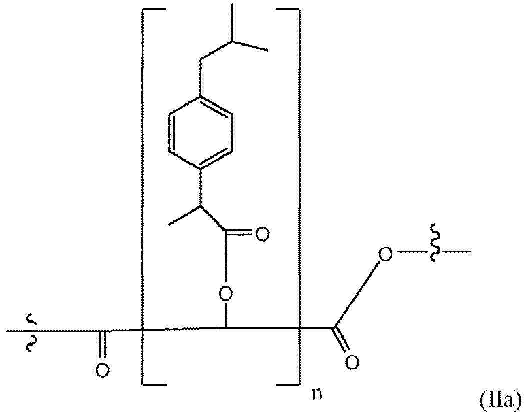 dans laquelle n vaut 1, 2, 3, 4, 5, 6, 7, 8 ou 9.

7. Polymère d'anhydride selon la revendication 4 qui comprend un ou plusieurs groupes de formule (IIb) : 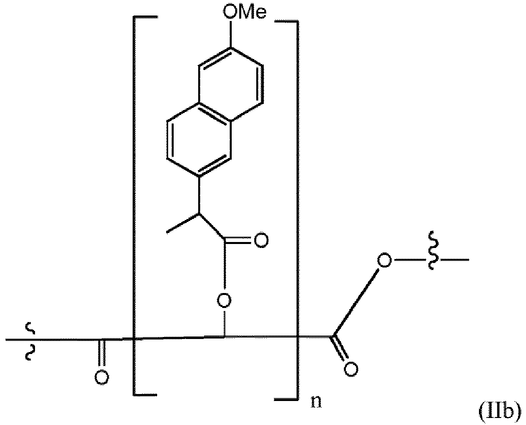 dans laquelle n vaut 1, 2, 3, 4, 5, 6, 7, 8 ou 9.

8. Polymère d'anhydride selon la revendication 6 ou 7 qui comprend 2 à 200 groupes répétés de formule (IIa) ou (IIb).

9. Polymère d'anhydride selon la revendication 6 ou 7 qui comprend au moins 2, 3, 4, 5, 6, 7, 8 ou 9 groupes répétés de formule (IIa) ou (IIb).

10. Polymère d'anhydride selon l'une quelconque des revendications 1 à 4 dans lequel chaque anti-inflammatoire non stéroïdien est choisi parmi l'ibuprofène, le naproxène, le fénoprofène, le kétoprofène, le flurbiprofène, le suprofène, le bénoxaprofène, l'indoprofène, le pirprofène, le carprofène, le loxoprofène, le pranoprofène, l'alminoprofène, l'acide salicylique, le diflusinal, le salsalate, l'oxaprozine, l'indométhacine, le sulindac, l'étodolac, le kétorolac, le diclofénac, le piroxicam, le méloxicam, le ténoxican, le lornoxicam, l'isoxicam, l'acide méfénamique, l'acide méclofénamique, l'acide flufénamique, l'acide tolfénamique, le lumiracoxib et la licofélone.

11. Polymère d'anhydride selon l'une quelconque des revendications 1 à 10 dans lequel le squelette comprend un ou plusieurs groupes de formule (V) :

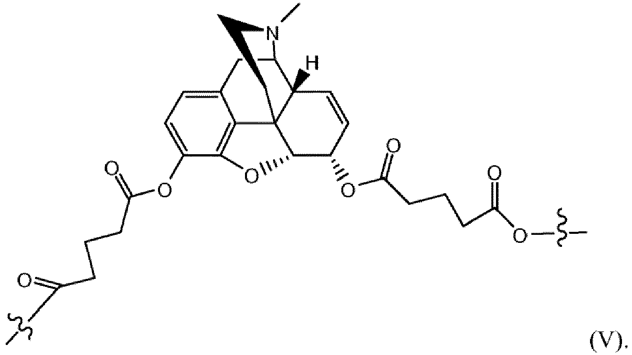

12. Microsphère qui comprend un polymère d'anhydride selon l'une quelconque des revendications 1 à 11.

13. Composition pharmaceutique comprenant un polymère d'anhydride tel que décrit dans l'une quelconque des revendications 1 à 12 et un véhicule pharmaceutiquement acceptable.

14. Polymère d'anhydride tel que décrit dans l'une quelconque des revendications 1 à 12 pour l'utilisation dans un traitement médical.

15. Polymère d'anhydride selon l'une quelconque des revendications 1 à 12 pour l'utilisation dans le traitement de la douleur.
